# EUROPÄISCHE PATENTSCHRIFT

(11) **EP 2 908 899 B1**
(45) Veröffentlichungstag und Bekanntmachung des Hinweises auf die Patenterteilung: **10.07.2019**
(21) Anmeldenummer: 13805740.1
(22) Anmeldetag: 18.10.2013
(51) Int. Cl.: A61M 25/00, A61M 29/00

(54) **MEDIZINISCHE VORRICHTUNG ZUM EINFÜHREN IN EINE KÖRPERÖFFNUNG ODER -HÖHLE EINES INDIVIDUUMS**
MEDICAL DEVICE FOR INTRODUCING INTO A BODILY ORIFICE OR CAVITY OF AN INDIVIDUAL
DISPOSITIF MÉDICAL DESTINÉ À ÊTRE INTRODUIT DANS UN ORIFICE OU UNE CAVITÉ DU CORPS D'UN INDIVIDU

(30) Priorität: 22.10.2012 DE 102012020693; 15.05.2013 DE 102013008316
(43) Veröffentlichungstag der Anmeldung: 26.08.2015
(73) Patentinhaber: Urotech GmbH, 83101 Rohrdorf-Achenmühle (DE)
(72) Erfinder: LAHME, Sven, 75175 Pforzheim (DE); PINKOWSKI, Wolfhard, 85609 Aschheim (DE); SCHWARZ, Werner, 83324 Ruhpolding (DE)
(74) Vertreter: Hofstetter, Schurack & Partner
(86) Internationale Anmeldenummer: PCT/DE2013/000618
(87) Internationale Veröffentlichungsnummer: WO 2014/063675

(56) Entgegenhaltungen:
- WO-A1-2011/084342
- DE-U1- 20 304 533
- US-A1- 2007 208 276
- US-A1- 2011 152 760

## Beschreibung

Die vorliegende Erfindung geht aus von einer medizinischen Vorrichtung zum Einführen in eine Körperöffnung oder -höhle eines Individuums, mit einem ersten länglichen Hohlkörper und einem zweiten länglichen Hohlkörper, in welchem der erste Hohlkörper aufgenommen ist, wobei zwischen einem Ende des zweiten Hohlkörpers, aus dem der erste Hohlkörper mit seinem zum Einführen in eine Körperöffnung oder -höhle eines Individuums vorgesehenen distalen Ende vorsteht, und diesem ersten Hohlkörper ein in radialer Richtung verlaufender stufenförmiger Übergang vorhanden ist, wobei das distale Ende des ersten Hohlkörpers als Spitzenteil ausgebildet ist oder ein Spitzenteil aufweist, welches bis zu dem zweiten Hohlkörper unter Bildung des stufenförmigen Übergangs verläuft.

Eine Vorrichtung der vorstehend genannten Art wird beispielsweise als medizinische Dilatatorvorrichtung und insbesondere als so genannter Ureter-Dilatator verwendet. Eine solche Dilatatorvorrichtung ist unter anderem in US 7.654.989 B2 und EP 1.173.248 B1 angegeben worden. Bei diesen bekannten Dilatatorvorrichtungen ist jedoch jeweils zwischen der Außenseite eines Dilatatortubus und der distalen Endseite eines den Dilatatortubus aufnehmenden Hüllrohres ein stufenförmiger Übergang gebildet. Dieser stufenförmige Übergang wird bei der Anwendung der betreffenden Dilatatorvorrichtung zuweilen als störend empfunden, da er zu Verletzungen bei Patienten führen kann, bei denen eine solche Vorrichtung zum Einsatz kommt. Es ist ferner eine medizinische Vorrichtung zum Einführen in eine Körperöffnung oder-höhle eines Individuums bekannt (WO 2011/084342 A1), die an ihrem distalen Einführungsende mehrere teleskopisch ausfahrbare Hohlkörper aufweist. Von diesen Hohlkörpern weist der jeweils auf der äußeren Seite der teleskopischen Anordnung liegende Hohlkörper an seinem distalen Ende einen stufenförmigen Übergang zur Außenfläche des von ihm aufgenommenen Hohlkörpers auf.

Zuweilen besteht jedoch zusätzlich der Wunsch, mittels einer Vorrichtung der vorstehend genannten Art auch eine Spülflüssigkeit in die Körperöffnung oder-höhle des Individuums abzugeben, in die die betreffende Vorrichtung einführbar oder eingeführt ist, und die Spülflüssigkeit aus der betreffenden Körperöffnung oder-höhle abzuführen.

Um eine Spülflüssigkeit von einer Dilatatorvorrichtung abgeben zu können, ist es bereits bekannt (siehe beispielsweise US 7.654.989 B2), die Dilatatorvorrichtung mindestens zweilumig auszubilden. Das eine Lumen der betreffenden Vorrichtung ist durch die Durchgangsöffnung eines Führungsrohres gebildet und dient zur Aufnahme des eigentlichen Dilatators. Ein zumindest weiteres Lumen befindet sich in einem auf der Außenseite des Führungsrohres vorgesehenen Zusatzrohr und dient zur Hindurchleitung einer Spülflüssigkeit. Damit weist aber eine solche zweilumige Dilatatorvorrichtung keinen gesonderten Rücklaufkanal für die Ableitung der in eine Körperöffnung oder-höhle eines Individuums eingeführten Spülflüssigkeit auf. Außerdem ist durch die Anordnung des Zusatzrohres auf der Außenseite des Führungsrohres die Außenabmessung der bekannten Dilatatorvorrichtung in unerwünschter Weise vergrößert. Es wäre zwar möglich, das Zusatzrohrlumen in den Außenumfang des Führungsrohrlumens zu integrieren. Dadurch würde jedoch das Innenlumen des Führungsrohres entweder bei unveränderter Führungsrohr-Außenabmessung in unerwünschter Weise kleiner werden, oder die Führungsrohr-Außenabmessung würde bei unverändertem Führungsrohr-Innenlumen in unerwünschter Weise vergrößert werden.

Der Erfindung liegt daher die Aufgabe zu Grunde, einen Weg zu zeigen, wie ausgehend von einer medizinischen Vorrichtung der eingangs genannten Art auf relativ einfache Weise der in radialer Richtung zwischen dem einen Ende des zweiten Hohlkörpers, aus dem der erste Hohlkörper vorsteht, und diesem ersten Hohlkörper verlaufende stufenförmige Übergang zumindest weitgehend beseitigt werden kann.

Außerdem soll eine medizinische Vorrichtung der eingangs genannten Art so weitergebildet werden, dass sie zusätzlich als Spülvorrichtung sowohl zur Abgabe einer Spülflüssigkeit in eine Körperöffnung oder-höhle eines Individuums als auch zur Ableitung der Spülflüssigkeit aus der betreffenden Körperöffnung oder -höhle nutzbar ist, ohne dass die Außenabmessung der Vorrichtung im Bereich ihrer Einführung in die Körperöffnung oder-höhle verändert werden muss.

Gelöst wird die vorstehend aufgezeigte Aufgabe ausgehend von einer medizinischen Vorrichtung der eingangs genannten Art gemäß der Erfindung zum einen dadurch, dass zwischen dem Spitzenteil und dem zweiten Hohlkörper eine in deren Längsrichtung verschiebbare Formungseinrichtung aufgenommen ist und dass die Formungseinrichtung im Bereich des stufenförmigen Übergangs so geformt ist, dass durch ihre Verschiebung in der betreffenden Längsrichtung das Spitzenteil zu dem stufenförmigen Übergang hin in radialer Richtung unter dessen zumindest weitgehender Aufhebung zu einem stufenlosen Übergang erweiterbar und/oder der zweite
Hohlkörper zu dem stufenförmigen Übergang hin in radialer Richtung unter dessen zumindest weitgehender Aufhebung zu einem stufenlosen Übergang zusammenziehbar ist.

Die Erfindung bringt den Vorteil mit sich, dass der in radialer Richtung zwischen dem einen Ende des zweiten Hohlkörpers, aus dem der erste Hohlkörper vorsteht, und diesem ersten Hohlkörper verlaufende stufenförmige Übergang auf relativ einfache Weise zumindest weitgehend beseitigt werden kann. Der zunächst stufenförmig gestaltete Übergang ist durch die vorliegende Erfindung in vorteilhafter Weise also in einen weitgehend stufenlosen Übergang überführbar. Ein solcher stufenloser Übergang ist aber für eine problemlose Einführung der betreffenden Vorrichtung in verschiedenste Öffnungen erforderlich. Bei Einsatz der Vorrichtung gemäß der Erfindung als medizinische Vorrichtung, wie als Ureter-Dilatator zur Einführung in eine Körperöffnung oder-höhle eines Individuums ist somit in vorteilhafter Weise ein Vorrichtungseinsatz ohne eine Verletzungsgefahr ermöglicht.

Entsprechend einer zweckmäßigen Weiterbildung der medizinischen Vorrichtung gemäß der Erfindung enthält die Formungseinrichtung ein Formungsteil, das auf seiner Außenseite an seinem zu dem stufenförmigen Übergang zwischen dem Spitzenteil und dem zweiten Hohlkörper hin zu bewegenden Bereich einen Drückteil aufweist, durch den im angrenzenden Bereich zwischen dem Spitzenteil und dem zweiten Hohlkörper der die in radialer Richtung kleinere Außenabmessung aufweisende Spitzenteil bis zu der größeren Außenabmessung in radialer Richtung des zweiten Hohlkörpers aufweitbar ist. Hierdurch ergibt sich der Vorteil eines relativ geringen konstruktiven Aufwands, um den erwähnten stufenförmigen Übergang zwischen dem Spitzenteil und dem zweiten Hohlkörper in einen zumindest angenähert stufenlosen Übergang zu überführen.

Nach einer anderen zweckmäßigen Weiterbildung der medizinischen Vorrichtung gemäß der Erfindung enthält die Formungseinrichtung ein Formungsteil, das auf seiner Außenseite an seinem zu dem stufenförmigen Übergang zwischen dem Spitzenteil und dem zweiten Hohlkörper hin zu bewegenden Bereich einen Vertiefungsteil aufweist, durch den im angrenzenden Bereich zwischen dem Spitzenteil und dem zweiten Hohlkörper der die in radialer Richtung größere Außenabmessung aufweisende zweite Hohlkörper bis zu dem die kleinere Außenabmessung in radialer Richtung aufweisenden Spitzenteil absenkbar ist. Durch diese zweckmäßigen Weiterbildung, die entweder alternativ oder zusätzlich zu der zuerst angegebenen zweckmäßigen Weiterbildung der medizinischen Vorrichtung gemäß der Erfindung angewandt werden kann, ergibt sich ebenfalls der Vorteil eines relativ geringen konstruktiven Aufwands, um den erwähnten stufenförmigen Übergang zwischen dem Spitzenteil und dem zweiten Hohlkörper in einen zumindest angenähert stufenlosen Übergang zu überführen.

Vorzugsweise ist das Formungsteil in Längsrichtung der beiden Hohlkörper hohl ausgebildet. Dadurch kommt man mit einem besonders einfachen Formungsteil aus, das überdies in seiner Längsrichtung zylinderförmig ausgebildet sein kann.

Vorzugsweise sind auch die beiden Hohlkörper zylinderförmig ausgebildet. Dies bringt den Vorteil mit sich, dass mit relativ einfach herzustellenden Hohlkörpern ausgekommen werden kann.

Zweckmäßigerweise ist mit zumindest einem der beiden Hohlkörper und der Formungseinrichtung ein Schiebemechanismus verbunden, durch dessen Betätigung die Formungseinrichtung relativ zu zumindest einem der beiden Hohlkörper in der Hohlkörperlängsrichtung verschiebbar ist. Durch diese Maßnahme ergibt sich der Vorteil, dass mit einem relativ geringen konstruktiven Aufwand für die Längsverschiebung der Formungseinrichtung ausgekommen werden kann.

Vorzugsweise besteht der Schiebemechanismus aus einem mit dem einen Hohlkörper verbundenen Aufnahmeteil und einem mit der Formungseinrichtung verbundenen Anlageteil, welcher in oder an den Aufnahmeteil anlegbar und relativ zu diesem in der Hohlkörperlängsrichtung verschiebbar ist. Dadurch ergibt sich der Vorteil eines bequem zu bedienenden Schiebemechanismus sowohl hinsichtlich des Zusammenfügens als auch hinsichtlich des Trennens von Aufnahmeteil und Anlageteil.

Gemäß weiterer zweckmäßiger Ausgestaltung der Erfindung weist der Schiebemechanismus zwischen dem Aufnahmeteil und dem Anlageteil eine Verriegelungseinrichtung auf, die ein Verschieben des Formkörpers relativ zu zumindest einem der Hohlkörper erst nach oder im Zuge einer Verriegelung des Anlageteiles an oder in dem Aufnahmeteil ermöglicht. Hierdurch wird der Vorteil einer besonders sicheren Bedienbarkeit des Schiebemechanismus erreicht, wie dies weiter unten noch näher ersichtlich werden wird.

Vorzugsweise ist die medizinische Vorrichtung gemäß der Erfindung sowohl in ihrer Grundform als auch unter Einbeziehung der vorstehend aufgezeigten Ausgestaltungen und Weiterbildungen als medizinischer Dilatator ausgebildet, an dessen für ein Einführen in eine Körperöffnung oder-höhle eines Individuums dienenden distalen Ende als erster Hohlkörper ein mit dem Spitzenteil versehener oder verbundener Dilatatortubus vorgesehen ist, der von einem Außentubus als zweitem Hohlkörper aufgenommen ist, zwischen dem und dem Dilatatortubus die in Längsrichtung verschiebbare Formungseinrichtung aufgenommen ist. Hierdurch ergibt sich der Vorteil, dass von einem Individuum, in dessen für eine Dilatation vorgesehene Körperöffnung oder -höhle der betreffende, beispielsweise durch einen Ureter-Dilatator gebildete Dilatator eingeführt wird, dieses Einführen nicht zu Verletzungen bei dem betreffenden Individuum führen kann.

An Hand von Zeichnungen wird die Erfindung nachstehend zunächst an einem konkreten Ausführungsbeispiel einer medizinischen Vorrichtung gemäß der Erfindung und danach unter Bezugnahme auf mehrere von möglichen Alternativlösungen zur Überführung eines zwischen zwei aneinander angrenzenden hohlförmigen Elementen bei einer solchen medizinischen Vorrichtung vorhandenen stufenförmigen Übergangs in einen zumindest weitgehend stufenlosen Übergang erläutert.

Gelöst wird die vorstehend genannte Aufgabe bei einer medizinischen Vorrichtung der eingangs genannten Art erfindungsgemäß zum einen dadurch, dass der erste längliche Hohlkörper und die Formungseinrichtung durch einen länglichen dritten Hohlkörper mit einer solchen Außenabmessung ersetzbar sind, dass zwischen dessen Außenseite und der Innenseite des ersten Hohlkörpers ein Zwischenraum gebildet ist, und dass dieser Zwischenraum bei Abgabe einer Spülflüssigkeit durch den dritten Hohlkörper in eine Körperöffnung oder-höhle eines Individuums als Rückflusskanal für aus der betreffenden Körperöffnung oder-höhle austretende gegebenenfalls Feststoffe mit sich führende Spülflüssigkeit nutzbar ist.

Durch die vorstehend angegebene Umbildung der eingangs genannten medizinischen Vorrichtung sind somit der erste Hohlkörper und die Formungseinrichtung durch einen dritten Hohlkörper ersetzt, der mit seinem Innenraum einen Spülkanal für die Abgabe einer Spülflüssigkeit in eine Körperöffnung oder-höhle eines Individuums bildet und der eine solche Außenabmessung aufweist, zwischen der und der Innenabmessung des ersten Hohlkörpers ein Zwischenraum gebildet ist. Dabei bildet dieser Zwischenraum bei Abgabe einer Spülflüssigkeit durch den dritten Hohlkörper einen Rückflusskanal für aus der betreffenden Körperöffnung oder -höhle austretende gegebenenfalls Feststoffe mit sich führende Spülflüssigkeit.

Die Erfindung bringt den Vorteil mit sich, dass die medizinische Vorrichtung der eingangs genannten Art auf relativ einfache Weise nicht nur zum Einführen in eine Körperöffnung oder-höhle eines Individuums, sondern auch als Spülvorrichtung zum Einleiten einer Spülflüssigkeit in die betreffende Körperöffnung oder-höhle und zugleich zum Ableiten, gegebenenfalls durch zusätzliches Absaugen der aus dieser Körperöffnung oder-höhle austretenden gegebenenfalls Feststoffe mit sich führenden Spülflüssigkeit verwendbar ist. Im Hinblick auf die Vorrichtung der eingangs genannten Art wird durch die Erfindung überdies die Außenabmessung dieser Vorrichtung im Bereich ihrer Einführung in die Körperöffnung oder -höhle nicht verändert; vielmehr bleibt die Außenabmessung der Vorrichtung im Bereich ihrer Einführung in die Körperöffnung oder-höhle unverändert erhalten.

Zweckmäßigerweise ist mit zumindest einem der den zweiten Hohlkörper und den dritten Hohlkörper umfassenden Hohlkörper ein Schiebemechanismus verbunden, durch dessen Betätigung der dritte Hohlkörper nach seinem Einführen in den zweiten Hohlkörper in dessen Längsrichtung verschiebbar ist. Durch diese Maßnahme ergibt sich der Vorteil, dass mit einem relativ geringen konstruktiven Aufwand für die Längsverschiebung des dritten Hohlkörpers ausgekommen werden kann.

Gemäß weiterer Ausgestaltung der Erfindung besteht der Schiebemechanismus aus einem mit einem der den zweiten Hohlkörper und den dritten Hohlkörper umfassenden Hohlkörper verbundenen Aufnahmeteil und einem mit dem anderen Hohlkörper verbundenen Anlageteil, welcher von dem Aufnahmeteil aufnehmbar und relativ zu diesem in der Hohlkörperlängsrichtung verschiebbar ist. Hierdurch ergibt sich der Vorteil einer besonders bequemen Bedienbarkeit des Schiebemechanismus sowohl hinsichtlich des Zusammenfügens als auch hinsichtlich des Trennens von Aufnahmeteil und Anlageteil.

Vorzugsweise weist der Schiebemechanismus zwischen dem Aufnahmeteil und dem Anlageteil eine Verriegelungseinrichtung auf, die ein Verschieben des dritten Hohlkörpers relativ zu dem ersten Hohlkörper lediglich bis zu einer Verriegelung des Anlageteiles an oder in dem Aufnahmeteil ermöglicht. Hierdurch wird der Vorteil einer besonders sicheren Bedienbarkeit des Schiebemechanismus erreicht, wie dies weiter unten noch näher ersichtlich werden wird.

Gemäß weiterer Ausgestaltung der Erfindung ist die Verriegelungseinrichtung durch zumindest ein Rastelement in dem Anlageteil oder in dem Aufnahmeteil und durch zumindest eine Aufnahme in dem Aufnahmeteil oder in dem Anlageteil gebildet. Dies bringt den Vorteil einer relativ einfachen Verriegelungseinrichtung mit sich.

Gemäß einer noch weiteren zweckmäßigen Ausgestaltung der Erfindung ist die jeweilige Aufnahme so gestaltet, dass das von ihr aufgenommene Rastelement vorzugsweise nach Verriegelung von Aufnahmeteil und Anlageteil quer zur Vorrichtungslängsrichtung in der zugehörigen Aufnahme verschiebbar ist. Diese Maßnahme ist von ganz besonderem Vorteil, um den von dem zweiten Hohlkörper aufgenommenen dritten Hohlkörper mit seiner Außenseite an die Innenseite des zweiten Hohlkörpers heranzuführen und um damit den Zwischenraum zwischen einem Bereich seiner Außenseite und der diesem gegenüberliegenden Innenseite des zweiten Hohlkörpers einseitig zu vergrößern. Dies kann in dem Fall von Nutzen sein, dass die aus einer Körperöffnung oder -höhle eines Individuums austretende Spülflüssigkeit, die durch den dritten Hohlkörper in diese Körperöffnung oder -höhle eingeführt worden ist, größere Feststoffe zur Ableitung mit sich führt, welche bei konzentrischer Anordnung des dritten Hohlkörpers in dem zweiten Hohlkörper durch den dann vorhandenen konzentrischen Zwischenraum nicht abgeleitet werden könnten.

Zum anderen wird die der Erfindung zu Grunde liegende Aufgabe bei einer medizinischen Vorrichtung der eingangs genannten Art erfindungsgemäß dadurch gelöst, dass
- bei Bildung des Spitzenteiles durch einen am distalen Vorrichtungsende vorgesehenen starren Spitzenteil und einen sich daran zum proximalen Vorrichtungsende hin anschließenden radial aufweitbaren Übergangsteil, der durch Verschieben der Formungseinrichtung von einer ersten Stellung, in der er mit dem zweiten Hohlkörper den stufenförmigen Übergang bildet, zu einer zweiten Stellung aufweitbar ist, in der er mit dem zweiten Hohlkörper den stufenlosen Übergang bildet, und
- bei Vorhandensein eines Zwischenraumes zwischen der Außenseite der Formungseinrichtung und der Innenseite des zweiten Hohlkörpers der Innenraum der Formungseinrichtung nach Entfernen des ersten Hohlkörpers samt seines Spitzenteiles unter Freilegung des genannten Zwischenraumes zum distalen Vorrichtungsende hin als Spülkanal für die Abgabe einer Spülflüssigkeit in eine Körperöffnung oder -höhle eines Individuums und der genannte Zwischenraum als Rückflusskanal für aus der betreffenden Körperöffnung oder -höhle austretende gegebenenfalls Feststoffe mit sich führende Spülflüssigkeit nutzbar sind.

Durch diese gerade angegebene Umbildung der eingangs genannten medizinischen Vorrichtung bildet somit der Innenraum der Formungseinrichtung nach Entfernen des ersten Hohlkörpers samt seines Spitzenteiles unter Freilegung des genannten Zwischenraumes zum distalen Vorrichtungsende hin einen Spülkanal für die Abgabe einer Spülflüssigkeit in eine Körperöffnung oder -höhle eines Individuums. Der genannte Zwischenraum bildet einen Rückflusskanal für aus der betreffenden Körperöffnung oder -höhle austretende gegebenenfalls Feststoffe mit sich führende Spülflüssigkeit.

Auch hierdurch ergibt sich der Vorteil, dass die medizinische Vorrichtung der eingangs genannten Art auf relativ einfache Weise nicht nur zum Einführen in eine Körperöffnung oder -höhle eines Individuums zu nutzen ist, sondern auch als Spülvorrichtung zum Einleiten einer Spülflüssigkeit in die betreffende Körperöffnung oder-höhle und zugleich zum Ableiten, gegebenenfalls durch zusätzliches Absaugen der aus dieser Körperöffnung oder-höhle austretenden gegebenenfalls Feststoffe mit sich führenden Spülflüssigkeit verwendbar ist. Im Hinblick auf die Vorrichtung der eingangs genannten Art wird durch die gerade angegebene Lösung der der Erfindung zu Grunde liegenden Aufgabe überdies die Außenabmessung dieser Vorrichtung im Bereich ihrer Einführung in die Körperöffnung oder -höhle nicht verändert; vielmehr bleibt die Außenabmessung der Vorrichtung im Bereich ihrer Einführung in die Körperöffnung oder-höhle unverändert erhalten.

Zweckmäßigerweise ist bei der vorstehend gerade aufgezeigten Ausbildung der eingangs genannten medizinischen Vorrichtung der erste Hohlkörper am proximalen Vorrichtungsende mittels eines Verriegelungsmechanismus lösbar festgehalten. Dies bringt den Vorteil sowohl einer besonders einfachen Sicherung des ersten Hohlkörpers und des von diesem getragenen Spitzenteiles in dem zweiten Hohlkörper als auch einer einfachen Freigabe des ersten Hohlkörpers und des von diesem getragenen Spitzenteiles aus dem zweiten Hohlkörper mit sich.

Vorzugsweise ist der vorstehend genannte Verriegelungsmechanismus in einem Anlageteil enthalten, welches von einem den zweiten Hohlkörper tragenden Aufnahmeteil aufnehmbar ist. Dadurch lässt sich der Verriegelungsmechanismus in vorteilhafter Weise mit besonders geringem konstruktiven Aufwand realisieren.

Zum weiteren wird die der Erfindung zu Grunde liegende Aufgabe bei einer medizinischen Vorrichtung der eingangs genannten Art erfindungsgemäß dadurch gelöst, dass
- bei Bildung des Spitzenteiles durch einen am distalen Vorrichtungsende vorgesehenen starren Spitzenteil und einen sich daran zum proximalen Vorrichtungsende hin anschließenden radial aufweitbaren Übergangsteil, der durch Verschieben der Formungseinrichtung von einer ersten Stellung, in der er mit dem zweiten Hohlkörper den stufenförmigen Übergang bildet, zu einer zweiten Stellung aufweitbar ist, in der er mit dem zweiten Hohlkörper den stufenlosen Übergang bildet, und
- bei Vorhandensein eines Zwischenraumes zwischen der Außenseite des Rohrteiles und der Innenseite des zweiten Hohlkörpers
   der Innenraum des ersten Hohlkörpers nach Entfernen allein des Spitzenteiles unter Freilegung des genannten Zwischenraumes zum distalen Vorrichtungsende hin als Spülkanal für die Abgabe einer Spülflüssigkeit in eine Körperöffnung oder-höhle eines Individuums und der genannte Zwischenraum als Rückflusskanal für aus der betreffenden Körperöffnung oder-höhle austretende gegebenenfalls Feststoffe mit sich führende Spülflüssigkeit nutzbar sind.

Durch diese gerade angegebene Umbildung der eingangs genannten medizinischen Vorrichtung bildet somit der Innenraum des ersten Hohlkörpers nach Entfernen allein des Spitzenteiles unter Freilegung des genannten Zwischenraumes zum distalen Vorrichtungsende hin einen Spülkanal für die Abgabe einer Spülflüssigkeit in eine Körperöffnung oder-höhle eines Individuums, und der genannte Zwischenraum bildet einen Rückflusskanal für aus der betreffenden Körperöffnung oder-höhle austretende gegebenenfalls Feststoffe mit sich führende Spülflüssigkeit.

Auch durch diese Umbildung der eingangs genannten medizinischen Vorrichtung ergibt sich der Vorteil, dass diese medizinische Vorrichtung auf relativ einfache Weise nicht nur zum Einführen in eine Körperöffnung oder -höhle eines Individuums zu nutzen ist, sondern auch als Spülvorrichtung zum Einleiten einer Spülflüssigkeit in die betreffende Körperöffnung oder -höhle und zugleich zum Ableiten, gegebenenfalls durch zusätzliches Absaugen der aus dieser Körperöffnung oder-höhle austretenden gegebenenfalls Feststoffe mit sich führenden Spülflüssigkeit verwendbar ist. Im Hinblick auf die Vorrichtung der eingangs genannten Art wird durch die gerade angegebene Lösung der der Erfindung zu Grunde liegenden Aufgabe überdies die Außenabmessung dieser Vorrichtung im Bereich ihrer Einführung in die Körperöffnung oder -höhle nicht verändert; vielmehr bleibt die Außenabmessung der Vorrichtung im Bereich ihrer Einführung in die Körperöffnung oder -höhle unverändert erhalten.

Vorzugsweise ist bei der vorstehend gerade aufgezeigten Ausbildung der eingangs genannten medizinischen Vorrichtung das Spitzenteil mit dem ersten Hohlkörper durch eine lösbare Verbindungseinrichtung verbunden. Hierdurch ergibt sich der Vorteil einer besonders einfachen Möglichkeit des Trennens des Spitzenteiles von dem ersten Hohlkörper.

Vorzugsweise ist die vorstehend genannte lösbare Verbindungseinrichtung durch eine Schraubverbindung gebildet. Dadurch steht in vorteilhafter Weise eine für die Verbindung von Spitzenteil und erstem Hohlkörper einfache und zugleich sichere Verbindungseinrichtung zur Verfügung, die eine leichte Trennung des Spitzenteiles von dem ersten Hohlkörper ermöglicht.
- Fig. 1: zeigt in einer nicht maßstäblichen Perspektivdarstellung ein Ausführungsbeispiel einer medizinischen Vorrichtung gemäß der Erfindung in Form eines Ureter-Dilatators.
- Fig. 2: zeigt in einer nicht maßstäblichen Schnittansicht die in Fig. 1 dargestellte medizinische Vorrichtung gemäß der Erfindung im nicht zusammengefügten Zustand.
- Fig. 3: zeigt in einer nicht maßstäblichen Schnittansicht die in Fig. 2 dargestellte medizinische Vorrichtung gemäß der Erfindung im zusammengefügten Zustand.
- Fig. 4: zeigt in einer stark vergrößerten Schnittansicht den zum Einführen der in Fig. 1 bis 3 dargestellten medizinischen Vorrichtung in eine Körperöffnung oder-höhle eines Individuums vorgesehenen distalen Bereich in einem Zustand, in welchem zwischen zwei aneinander angrenzenden hohlförmigen Elementen ein stufenförmiger Übergang existiert.
- Fig. 5: zeigt in einer stark vergrößerten Schnittansicht den zum Einführen der in Fig. 1 bis 3 dargestellten medizinischen Vorrichtung in eine Körperöffnung oder-höhle eines Individuums vorgesehenen distalen Bereich in einem Zustand, in welchem zwischen zwei aneinander angrenzenden hohlförmigen Elementen der in Fig. 4 dargestellte stufenförmige Übergang in einen zumindest weitgehend stufenlosen Übergang überführt ist.
- Fig. 6 und 7: zeigen eine Alternativlösung zu der in Fig. 4 und 5 gezeigten Lösung für die Überführung eines stufenförmigen Übergangs zwischen zwei aneinander angrenzenden hohlförmigen Elementen in einen zumindest weitgehend stufenlosen Übergang.
- Fig. 8 und 9: zeigen eine weitere Alternativlösung zu der in Fig. 4 und 5 gezeigten Lösung für die Überführung eines stufenförmigen Übergangs zwischen zwei aneinander angrenzenden hohlförmigen Elementen in einen zumindest weitgehend stufenlosen Übergang.
- Fig. 10 und 11: zeigen eine noch weitere Alternativlösung zu der in Fig. 4 und 5 gezeigten Lösung für die Überführung eines stufenförmigen Übergangs zwischen zwei aneinander angrenzenden hohlförmigen Elementen in einen zumindest weitgehend stufenlosen Übergang.
- Fig. 12: zeigt in einer nicht maßstäblichen Perspektivdarstellung den zweiten länglichen Hohlkörper der medizinischen Vorrichtung gemäß der Erfindung, nachdem deren erster länglicher Hohlkörper und die Formungseinrichtung aus diesem zweiten Hohlkörper entfernt worden sind.
- Fig. 13: zeigt in einer nicht maßstäblichen Perspektivdarstellung einen dritten länglichen Hohlkörper, der in einer ersten Weiterbildung der medizinischen Vorrichtung gemäß der Erfindung in den in Fig. 1 dargestellten zweiten Hohlkörper der medizinische Vorrichtung unter Bildung einer Spülvorrichtung einzuführen ist.
- Fig. 14: zeigt in einer nicht maßstäblichen Perspektivdarstellung die medizinische Vorrichtung mit in den zweiten Hohlkörper gemäß Fig. 13 eingesetztem dritten Hohlkörper gemäß Fig. 13.
- Fig. 15: zeigt eine perspektivische Schnittansicht der in Fig. 14 dargestellten medizinischen Vorrichtung.
- Fig. 16: zeigt eine Draufsicht auf das in Fig. 14 rechts dargestellte proximale Ende der medizinischen Vorrichtung.
- Fig. 17: zeigt in einer nicht maßstäblichen Schnittansicht den distalen Endbereich der medizinischen Vorrichtung gemäß der Erfindung in einer das Spitzenteil und die Formungseinrichtung umfassenden solchen Ausgestaltung, die für die Bildung einer Spülvorrichtung gemäß der vorliegenden Erfindung geeignet ist, wobei sich die Formungseinrichtung in einer ersten Einstellposition befindet.
- Fig. 18: zeigt den in Fig. 17 dargestellten distalen Endbereich der medizinischen Vorrichtung in einer nicht maßstäblichen Perspektivdarstellung.
- Fig. 19: zeigt in einer nicht maßstäblichen Schnittansicht die in Fig. 17 dargestellte Ausgestaltung des distalen Endbereichs der medizinischen Vorrichtung, wobei sich die Formungseinrichtung in einer von der ersten Einstellposition verschiedenen zweiten Einstellposition befindet.
- Fig. 20: zeigt den in Fig. 19 dargestellten distalen Endbereich der medizinischen Vorrichtung in einer nicht maßstäblichen Perspektivdarstellung.
- Fig. 21: zeigt in einer nicht maßstäblichen Schnittansicht den in Fig. 17 bis 20 dargestellten distalen Endbereich der medizinischen Vorrichtung nach Umbildung der betreffenden Vorrichtung zu einer Spülvorrichtung gemäß der vorliegenden Erfindung.
- Fig. 22: zeigt in einer nicht maßstäblichen Schnittansicht einen proximalen Anlageteil der medizinischen Vorrichtung gemäß der Erfindung, in welchem der erste Hohlkörper und die Formungseinrichtung aufgenommen sind, die den in Fig. 17 bis 20 dargestellten distalen Endbereich der medizinischen Vorrichtung aufweisen.
- Fig. 23: zeigt in einer nicht maßstäblichen Schnittansicht den distalen Endbereich des ersten Hohlkörpers und der Formungseinrichtung gemäß Fig. 22 in Verbindung mit einem Spitzenteil.
- Fig. 24: zeigt in einer nicht maßstäblichen Schnittansicht einen gegenüber dem in Fig. 17 dargestellten distalen Endbereich der medizinischen Vorrichtung gemäß der Erfindung modifizierten distalen Endbereich, bei dem das Spitzenteil und der erste Hohlkörper durch eine lösbare Verbindungseinrichtung miteinander verbunden sind, wobei sich die Formungseinrichtung in einer ersten Stellung bzw. Einstellposition befindet.
- Fig. 25: zeigt den in Fig. 24 dargestellten modifizierten distalen Endbereich, wobei sich die Formungseinrichtung in einer ersten Stellung verschiedenen zweiten Stellung bzw. Einstellposition befindet,
- Fig. 26: zeigt in einer nicht maßstäblichen Schnittansicht den in Fig. 24 und 25 dargestellten distalen Endbereich der medizinischen Vorrichtung nach Umbildung der betreffenden Vorrichtung zu einer Spülvorrichtung gemäß der vorliegenden Erfindung.
- Fig. 27: zeigt in einer nicht maßstäblichen Perspektivdarstellung das in Fig. 1 gezeigte Ausführungsbeispiel einer medizinischen Vorrichtung gemäß der Erfindung, bei der auf den zweiten Hohlkörper ein Versteifungshohlkörper aufgebracht ist.
- Fig. 28: zeigt in einer nicht maßstäblichen Schnittansicht den distalen Endbereich des zweiten Hohlkörpers in Verbindung mit einem Spitzenteil.
- Fig. 29: zeigt in einer vergrößerten Schnittansicht eine mögliche Querschnittsform des Versteifungshohlkörpers.
- Fig. 30: zeigt in einer vergrößerten Schnittansicht eine alternative Querschnittsform des Versteifungshohlkörpers.

Bevor auf die Zeichnungen näher eingegangen wird, sei zunächst angemerkt, dass entsprechende Teile bzw. Elemente in sämtlichen Zeichnungsfiguren durch gleiche Bezugszeichen bezeichnet sind.

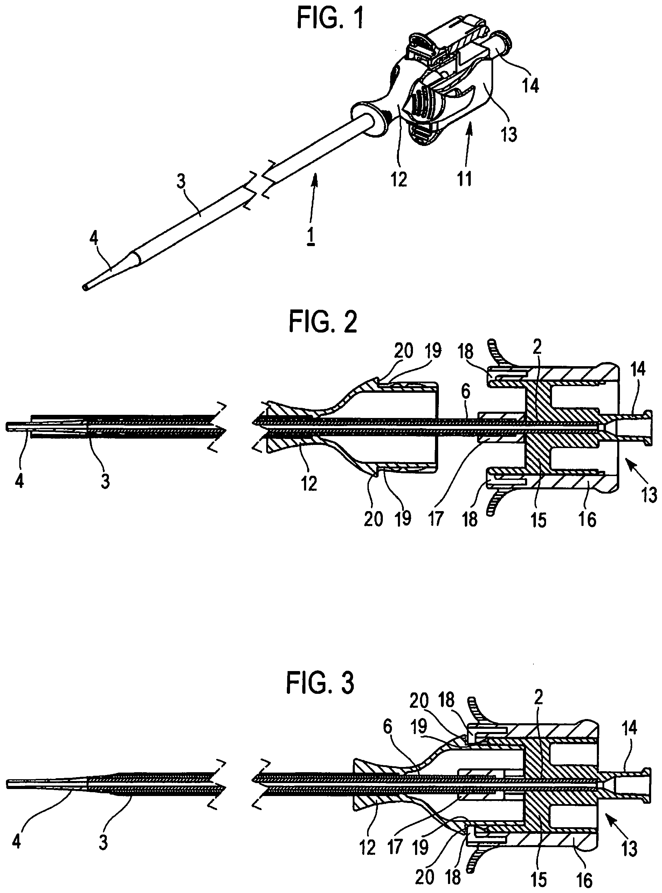

In Fig. 1 bis 5 ist als ein Ausführungsbeispiel der Erfindung in einer nicht maßstäblichen Perspektivdarstellung eine medizinische Vorrichtung 1 gemäß der Erfindung in Form eines Dilatators 1 gezeigt. Dieser Dilatator 1, der insbesondere ein Ureter-Dilatator sein kann, dient zum Einführen in eine (nicht dargestellte) Körperöffnung oder -höhle eines Individuums und weist einen in Fig. 2 bis 5 näher gezeigten ersten länglichen Hohlkörper 2 und einen zweiten länglichen Hohlkörper 3 auf, in welchem der erste Hohlkörper 2 aufgenommen ist.

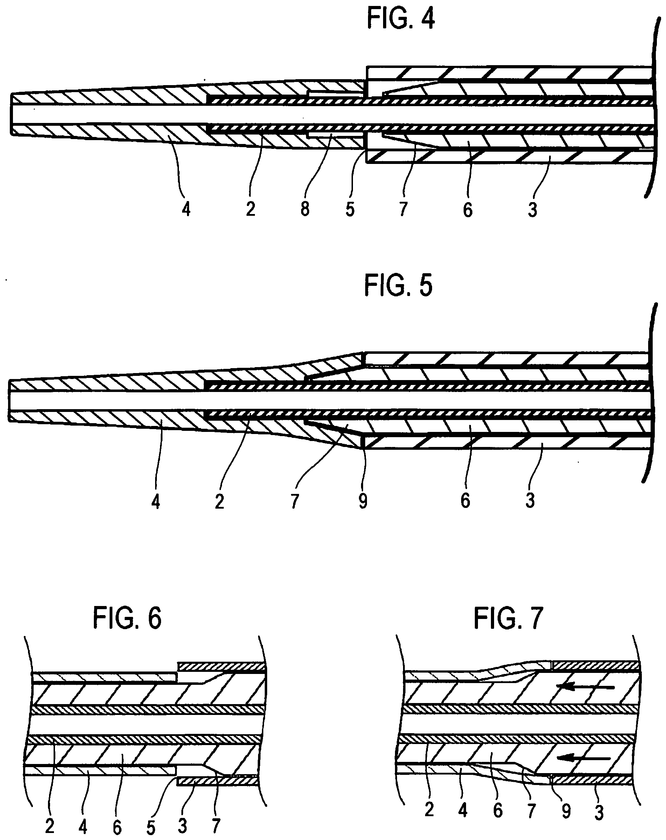

Die beiden Hohlkörper 2 und 3 des Dilatators 1 sind hier zylinderförmig ausgebildet, also jeweils durch einen Tubus gebildet. Der erste Hohlkörper 2, der einen Dilatatortubus 2 bildet, ist an seinem distalen Ende - das ist das in Fig. 1 links darstellte Ende - als Spitzenteil 4 ausgebildet oder weist ein solches gesondertes Spitzenteil 4 auf, wie dies aus Fig. 2 bis 5 näher ersichtlich ist. Dieses auf seiner Außenlängsseite kegelförmig oder kegelstumpfförmig ausgebildete Spitzenteil 4 verläuft bis zu dem einen Außentubus darstellenden zweiten Hohlkörper 3 unter Bildung des bereits erwähnten stufenförmigen Übergangs 5 (siehe Fig. 4). Das Spitzenteil 4 ist somit gewissermaßen Teil des ersten Hohlkörpers 2, der - anders gesagt - aus einem länglichen zylinderförmigen Teil und dem Spitzenteil 4 besteht.

Zwischen dem distalen Ende des zweiten Hohlkörpers 3, aus dem der erste Hohlkörper 2 mit seinem zum Einführen in eine Körperöffnung oder-höhle eines Individuums dienenden Spitzenteil 4 vorsteht, und diesem Spitzenteil 4 ist zunächst ein in radialer Richtung verlaufender stufenförmiger Übergang 5 vorhanden, wie er aus der stark vergrößerten Schnittansicht von Fig. 4 ersichtlich ist.

An dieser Stelle sei angemerkt, dass der äußere Hohlkörper 3 des Dilatators 1 in dem Fall, dass er als zylinderförmiger Tubus ausgeführt ist, bei seiner tatsächlichen Ausführung einen Durchmesser von etwa 2mm bis 6mm und eine Länge von etwa 5cm bis 100cm aufweisen wird.

Zwischen dem Spitzenteil 4 und dem zweiten Hohlkörper 3 bzw. Außentubus 3 ist, wie dies aus Fig. 2 bis 5 hervorgeht, eine in dessen Längsrichtung verschiebbare Formungseinrichtung aufgenommen, die gemäß Fig. 4 und 5 aus einem Formungsteil 6 und einem Drückteil 7 besteht. Das Formungsteil 6 und das Drückteil 7 können, wie im vorliegenden Fall, zusammenhängend durch ein einziges zylinderförmiges Teil mit einer als Drückglied wirkenden kegelstumpfförmigen Spitze gebildet sein.

Das Drückteil 7 der Formungseinrichtung ist, wie dies aus Fig. 4 und 5 näher ersichtlich ist, im Bereich des stufenförmigen Übergangs 5 so geformt, dass durch seine Verschiebung in der betreffenden Längsrichtung das Spitzenteil 4 an dem stufenförmigen Übergang in radialer Richtung unter zumindest weitgehender Aufhebung dieses stufenförmigen Übergangs 5 zu einem stufenlosen Übergang 9 erweiterbar ist. Das hier kegelförmig oder kegelstumpfförmig ausgebildete Drückteil 7 wird im Zuge seiner erwähnten Längsverschiebung in einen Aufnahmeraum 8 des Spitzenteiles 4 hinein geschoben, der nach erfolgter Längsverschiebung zumindest weitgehend durch das Drückteil 7 ausgefüllt ist. Wie aus Fig. 2 bis 5 ersichtlich ist, sind bei dem vorliegenden Dilatator 1 das Formungsteil 6 und das Drückteil jeweils hohl und insbesondere zylinderförmig ausgebildet. Der Spitzenteil 4 wird dabei zumindest im Bereich des erwähnten stufenförmigen Übergangs 5 wie auch der Außentubus 3 und die Formungseinrichtung (6, 7) aus einem Schlauchmaterial, wie einem zähelastischen Kunststoffschlauch bestehen; im Falle des Spitzenteiles 4 kann dieser Kunststoffschlauch in seiner radialen Richtung durch das Drückteil 7 der Formungseinrichtung aufgeweitet werden, und er kann sich nach dessen Zurückziehen in seiner radialen Richtung wieder zusammenziehen bzw. verengen. Die für die medizinische Vorrichtung 1 zu verwendenden Materialien müssen natürlich biokompatibel sein, soweit sie mit Gewebe einer Körperöffnung oder-höhle eines Individuums in Kontakt gelangen, in die die betreffende Vorrichtung eingeführt wird.

Um die zuvor erwähnte Aufweitung des Spitzenteiles 4 zu erleichtern, kann dieses in seiner Längsrichtung geschlitzt sein, vorzugsweise durch einige, wie zum Beispiel durch vier Schlitze in seinem an den erwähnten stufenförmigen Übergang angrenzenden Bereich. Diese Schlitzung kann bei einer tatsächlichen Ausführungsform eine Länge im Bereich von beispielsweise 2 bis 20mm aufweisen.

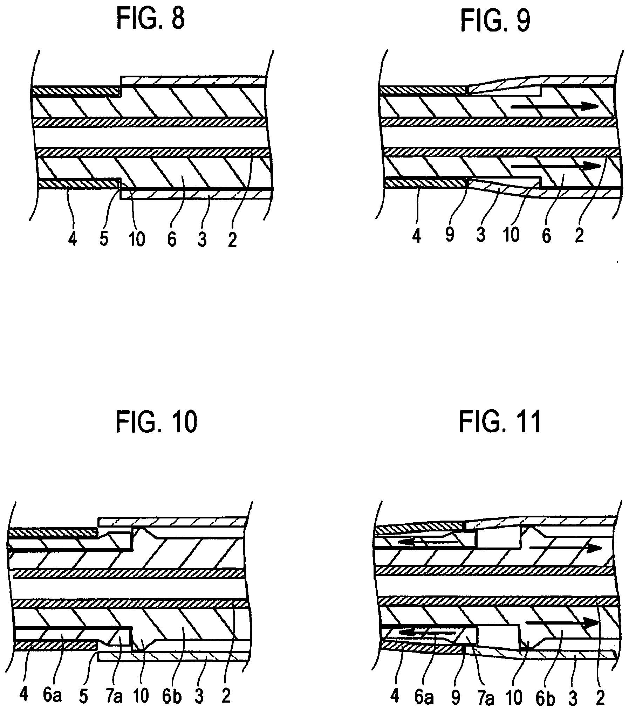

Es sei jedoch an dieser Stelle angemerkt, dass die Formungseinrichtung alternativ oder zusätzlich zu der zuvor betrachteten Ausführungsform im Bereich des stufenförmigen Übergangs 5 auch so geformt sein kann, dass der den zweiten Hohlkörper 3 bildende Außentubus 3 an dem stufenförmigen Übergang in radialer Richtung unter zumindest weitgehender Aufhebung des stufenförmigen Übergangs in einen stufenlosen Übergang verengbar bzw. zusammenziehbar ist. Hierauf wird weiter unten unter Bezugnahme auf die Fig. 8 bis 11 noch näher eingegangen. Dabei wird in einem solchen Fall der betreffende Außentubus 3 zumindest im Bereich des erwähnten stufenförmigen Übergangs 5 aus einem Schlauchmaterial, wie einem zähelastischen Kunststoffschlauch bestehen, der in seiner radialen Richtung durch die Formungseinrichtung aufgeweitet werden kann und der sich nach deren Zurückziehen in seiner radialen Richtung zusammenzieht bzw. verengt.

Bevor auf weitere Details in Fig. 1 bis 3 näher eingegangen wird, seien zunächst in Fig. 6 bis 11 dargestellte Alternativlösungen für die Überführung des stufenförmigen Übergangs 5 in einen stufenlosen Übergang 9 betrachtet. Auch bei diesen Alternativlösungen wird davon ausgegangen, dass die jeweilige Formungseinrichtung sowie der jeweilige Außentubus 3 und/oder das Spitzenteil 4 aus einem Schlauchmaterial, wie einem zähelastischen Kunststoffschlauch bestehen. Dieser Kunststoffschlauch kann im Falle des Außentubus 3 und/oder des Spitzenteiles 4 zumindest im Bereich des erwähnten stufenförmigen Übergangs 5 in seiner radialen Richtung durch die Formungseinrichtung aufgeweitet werden, und er kann sich nach deren Zurückziehen in seiner radialen Richtung wieder zusammenziehen bzw. verengen.

Ganz allgemein können bei jeder Ausführungsform der erfindungsgemäßen Vorrichtung der zweite Hohlkörper bzw. Außentubus 3 und/oder die Formungseinrichtung aus einer hohlen elastischen Längsspirale bestehen, die aus einem Metall oder Kunststoff hergestellt ist. Mittels solcher flexibler Längsspiralen lässt sich die erfindungsgemäße Vorrichtung relativ bequem durch in unterschiedlichen Richtungen verlaufende Körperöffnungen einführen, an deren Verläufe sich die Längsspiralen leicht anpassen können. Nach einem Zurückziehen der betreffenden Vorrichtung aus solchen Körperöffnungen können die Längsspiralen dann ihre beispielsweise gestreckten Ausgangsformen wieder leicht annehmen.

Die Fig. 6 und 7 zeigen eine erste Alternativlösung zur Überführung des erwähnten stufenförmigen Übergangs 5 in einen zumindest weitgehend stufenlosen Übergang 9. Dabei ist hier lediglich der Bereich um den betreffenden Übergang herum in stark vergrößertem Maßstab dargestellt. Fig. 6 zeigt den Zustand vor einer Längsverschiebung des Formungsteiles 6 mit seinem Drückteil 7, und Fig. 7 zeigt den Zustand nach der in Richtung der dort angegebenen Pfeile erfolgten Längsverschiebung. Es erfolgt hier also eine entsprechende Aufweitung des Spitzenteiles 4 im Bereich des stufenförmigen Übergangs 5 wie bei der in Fig. 2 bis 5 gezeigten Ausführungsform.

In Fig. 8 und 9 ist eine weitere Alternativlösung zur Überführung des erwähnten stufenförmigen Übergangs 5 in einen zumindest weitgehend stufenlosen Übergang 9 gezeigt. Dabei ist auch hier lediglich der Bereich um den betreffenden Übergang herum in stark vergrößertem Maßstab dargestellt. Im Unterschied zu der zuvor betrachteten Alternativlösung weist gemäß Fig. 8 und 9 die Formungseinrichtung an dem Formungsteil 6 ein Vertiefungsteil 10 in Form eines Absatzes auf. Dieses Vertiefungsteil 10 gibt auf eine Längsverschiebung des Formungsteiles 6 in Richtung der in Fig. 9 angegebenen Pfeile die Unterstützung des Außentubus in der radialen Richtung frei, der sich sodann in der radialen Richtung zusammenziehen kann und mit dem Spitzenteil 4 den stufenlosen Übergang 9 bildet.

In Fig. 10 und 11 ist eine noch weitere Alternativlösung zur Überführung des erwähnten stufenförmigen Übergangs 5 in einen zumindest weitgehend stufenlosen Übergang 9 dargestellt. Während bei den zuvor betrachteten Konstruktionen zur Überführung des erwähnten stufenförmigen Übergangs 5 in einen zumindest weitgehend stufenlosen Übergang 9 das Formungsteil 6 der Formungseinrichtung jeweils nur in einer Längsrichtung zu verschieben ist, zeigen die Fig. 10 und 11 eine Vorrichtung, bei der die Formungseinrichtung sowohl ein Formungsteil 6a mit einem Drückteil 7a als auch ein Formungsteil 6b mit einem Vertiefungsteil 10 umfasst. Zur Überführung des zwischen dem Außentubus 3 und dem Spitzenteil 4 zunächst vorhandenen stufenförmigen Übergangs 5 in einen zumindest angenähert stufenlosen Übergang 9 werden die beiden Formungsteile 6a und 6b von der in Fig. 10 gezeigten Position aus in entgegengesetzte Richtungen voneinander weg in Längsrichtung verschoben, wie dies die in Fig. 11 eingetragenen Pfeile erkennen lassen. Diese Verschiebungen erfolgen zweckmäßigerweise gleichzeitig.

Um die erwähnte Längsverschiebung der jeweiligen Formungseinrichtung und insbesondere des jeweiligen Formungsteiles 6 bzw. 6a, 6b vorzunehmen, ist gemäß der Erfindung ein Schiebemechanismus 11 vorgesehen, dessen mögliche Ausführungsform in Fig. 1 bis 3 dargestellt ist. Dieser in Fig. 1 zum Teil aufgeschnitten dargestellte Schiebemechanismus 11 besteht bei der vorliegenden Ausführungsform aus einem Aufnahmeteil 12 und einem Anlageteil 13.

Das Aufnahmeteil 12 ist mit einem der beiden Hohlköper 2 und 3, und zwar im vorliegende Fall mit dem den zweiten Hohlkörper 3 bildenden Außentubus 3 fest verbunden. Das Anlageteil 13, welches an dem Aufnahmeteil 12 in Achsrichtung der Vorrichtung 1 von deren in Fig. 1 bis 3 rechts dargestellter proximaler Seite her in Anlage bringbar und bei Bedarf von dem betreffenden Aufnahmeteil 12 wieder gelöst werden kann, besteht aus einem Innenteil 15 und einem Außenteil 16, von dem das Innenteil 15 in Achsrichtung der Vorrichtung 1 gleitbar aufgenommen ist. Das Innenteil 15 ist mit dem den ersten Hohlkörper 2 bildenden Dilatatortubus 2 fest verbunden. An seinem in Fig. 1 bis 3 rechts dargestellten proximalen Ende ist das Innenteil 15 mit einem Anschlussteil 14 versehen, an dem verschiedene medizinische Geräte, wie zum Beispiel Spritzen, angeschlossen werden können. Das betreffende Anschlussteil kann beispielsweise ein so genanntes Luer-Anschlussteil sein.

Das zuvor erwähnte Außenteil 16 ist mittels eines den Dilatatortubus 2 gleitbar aufnehmenden Gleitteiles 17 mit dem proximalen Ende des durch einen zylindrischen Tubus gebildeten Formungsteiles 6 fest verbunden. Dieses Gleitteil 17, welches dieselbe Schraffur trägt wie das Außenteil 16, ist über einen in Fig. 2 und 3 nicht sichtbaren Quersteg mit dem Außenteil 16 zusammenhängend verbunden. Der betreffende Quersteg verläuft dabei senkrecht zur Zeichenebene der Fig. 2 und 3. Somit ist mit dem einen der beiden Hohlkörper 2, 3 und der Formungseinrichtung bzw. mit deren Formungsteil 6 bzw. 6a, 6b der Schiebemechanismus 11 verbunden, durch dessen Betätigung die Formungseinrichtung relativ zumindest zu dem einen der beiden Hohlkörper 2, 3 in dessen Längsrichtung verschiebbar ist.

Der Schiebemechanismus 11 weist zwischen dem Aufnahmeteil 12 und dem Anlageteil 13 eine Verriegelungseinrichtung auf, die im vorliegenden Fall durch Rastnasen 18 an der Innenseite des Außenteiles 16 des Anlageteiles 13 und zu diesen Rastnasen 18 passende Rastnasenaufnahmen 19 am äußeren Umfang des Aufnahmeteiles 12 gebildet ist. Das Aufnahmeteil 12 weist außerdem Anschläge 20 in Form von Vorsprüngen auf, die ein Verschieben des gesamten Anlageteiles 13 in Vorrichtungslängsrichtung auf dem Aufnahmeteil 12 begrenzen. Durch diese Verriegelungseinrichtung kann die Formungseinrichtung mit ihrem jeweiligen Formungsteil 6 bzw. 6a und 6b relativ zu dem einen Hohlkörper erst nach oder im Zuge einer Verriegelung des Anlageteiles 13 an oder in dem Aufnahmeteil 12 verschoben werden.

Um die erwähnte Längsverschiebung der Formungseinrichtung bei der in Fig. 1 bis 3 dargestellten Ausführungsform ausführen zu können, wird dort der den ersten Hohlkörper bildende Innen- bzw. Dilatatortubus 2 zusammen mit der Formungseinrichtung vorzugsweise von der proximalen Vorrichtungsseite her durch den den zweiten Hohlkörper bildenden Außentubus 3 geschoben. Die Entnahme des Dilatatortubus 2 aus der Vorrichtung 1 erfolgt durch dessen Herausziehen aus dem Außentubus 3 zur proximalen Vorrichtungsseite hin.

Es sei an dieser Stelle angemerkt, dass die vorstehend erwähnte Verriegelungseinrichtung auch durch andere an dem Aufnahmeteil 12 und dem Anlegeteil 13 gebildete Verriegelungselement realisiert sein kann. Auch die Verschiebung des Anlegeteiles 13 unter Anlage an dem Aufnahmeteil 12 kann anders begrenzt sein als durch die hier in Form von Vorsprüngen vorliegenden Anschläge 20. So kann die betreffende Verschiebung entweder zusätzlich oder alternativ zu der erläuterten Begrenzung durch Anlagen des Anlageteiles 13 an einem anderen Umfangsbereich des Aufnahmeteiles 12 begrenzt werden.

Vorstehend ist ein medizinischer Dilatator 1 an Hand einer Ausführungsform der Erfindung sowie unter Bezugnahme auf einige Alternativlösungen erläutert worden. Für den in dem Dilatator 1 enthaltenen Dilatatortubus 2 mit seinem Spitzenteil 4 stellt der Außentubus 3 gewissermaßen eine Dilatatorschleuse dar, innerhalb der der Dilatatortubus und die Formungseinrichtung mit ihrem Formungsteil 6 oder Formungsteilen 6a, 6b und mit ihrem Drückteil 7 oder Vertiefungsteil 10 verschiebbar sind.

Bei der erläuterten Ausführungsform und auch bei den betrachteten Alternativlösungen ist davon ausgegangen worden, dass der in einen stufenlosen Übergang 9 zu überführende stufenförmige Übergang 5 durch einen Bereich kleinerer Abmessung eines Spitzenteiles 4 in radialer Richtung und einen an das Spitzenteil 4 angrenzenden zweiten Hohlkörper 3 mit demgegenüber größerer Abmessung in radialer Richtung gebildet ist. Der betreffende stufenförmige Übergang 5 stellt also eine Erweiterung der Außenabmessung der Vorrichtung 1 in deren radialer Richtung vom distalen Vorrichtungsende aus gesehen dar. Die vorliegende Erfindung ist jedoch auf die Überführung eines solchen stufenförmigen Übergangs 5 in einen stufenlosen Übergang 9 nicht beschränkt. So könnte der betreffende stufenförmige Übergang auch durch einen Bereich größerer Abmessung des Spitzenteiles 4 in radialer Richtung und den an das Spitzenteil 4 angrenzenden zweiten Hohlkörper 3 mit demgegenüber kleinerer Abmessung in radialer Richtung gebildet sein. In diesem Fall müsste die jeweilige Formungseinrichtung in ihrer Lage bezogen auf die in den Zeichnungsfiguren jeweils dargestellte Lage um 180° gewendet werden.

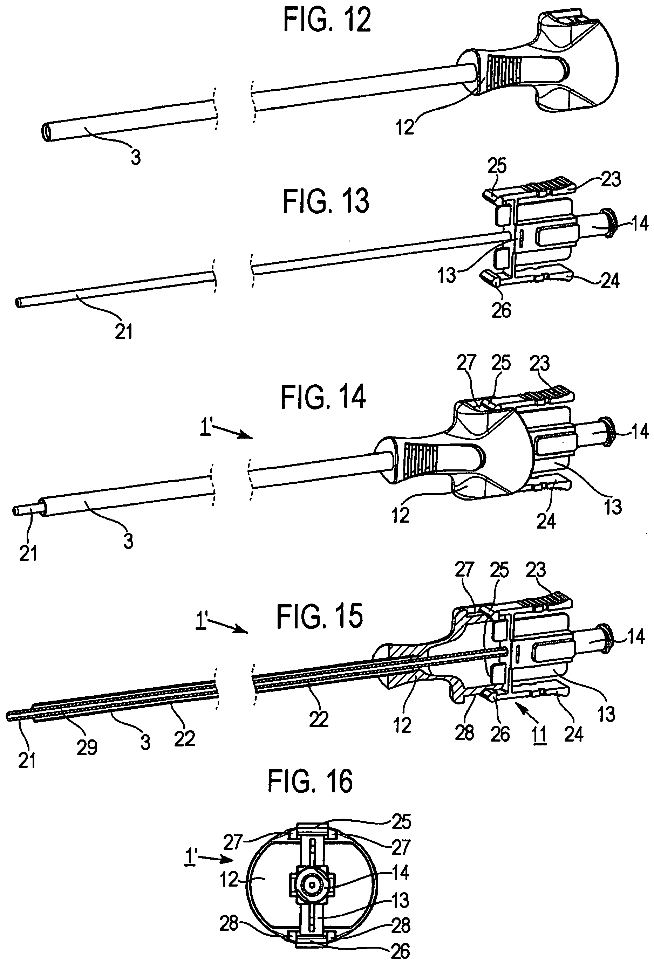

In Fig.12 ist von der medizinischen Vorrichtung gemäß dem Fig. 1 lediglich der äußere zweite längliche Hohlkörper 3 mit dem an seinem rechts dargestellten proximalen Ende mit ihm verbundenen, zu dem proximalen Ende hin trichterförmig erweiterten Aufnahmeteil 12 des in Fig. 1 dargestellten Schiebemechanismus 11 gezeigt. Gemäß Fig.12 sind also aus der medizinischen Vorrichtung gemäß Fig. 1 der dort vorgesehene erste Hohlkörper mit seinem Spitzenteil (in Fig. 2 und 3 mit 2 bzw. 4 bezeichnet) und die Formungseinrichtung (in Fig. 4 bis 11 mit 6 bezeichnet) aus dem zweiten Hohlkörper 3 entfernt worden. Der zweite Hohlkörper 3 ist auch hier durch ein Rohr mit einem durchgehenden Lumen in dessen Längsrichtung oder einen Schlauch gebildet, vorzugsweise aus einem biokompatiblen Kunststoff, wie beispielsweise Polyurethan.

Der Aufnahmeteil 12 ist zu seinem proximalen Ende hin trichterförmig erweitert, um - wie unten noch näher ersichtlich wird - einen Anlageteil 13 aufzunehmen. Dieser Anlageteil 13 bildet zusammen mit dem Aufnahmeteil 12 den erwähnten Schiebemechanismus 11.

In Fig. 13 ist ein weiterer länglicher Hohlkörper dargestellt, der in Bezug auf die medizinische Vorrichtung gemäß Fig. 1 bis 3 als dritter Hohlkörper 21 bezeichnet wird und durch den eine Spülflüssigkeit hindurchleitbar ist. Auch dieser dritte Hohlkörper 21 ist durch ein Rohr mit einem durchgehenden Lumen 29 in dessen Längsrichtung oder durch einen Schlauch gebildet, vorzugsweise ebenfalls aus einem biokompatiblen Kunststoff, wie beispielsweise Polyurethan. Das erwähnte Lumen 29 im Innern des dritten Hohlkörpers 21 legt, wie noch ersichtlich wird, einen Spülkanal der medizinischen Vorrichtung für die Abgabe einer Spülflüssigkeit in eine Körperöffnung bzw. -höhle eines Individuums fest, in die die betreffende Vorrichtung eingeführt ist.

Die Außenabmessung des dritten Hohlkörpers 21 rechtwinklig zu dessen Längsrichtung ist kleiner als die Innenabmessung des zweiten Hohlkörpers 2 rechtwinklig zu dessen Längsrichtung. In dem Fall, dass beide Hohlkörper 3 und 21 durch Rohre oder Schläuche gebildet sind, kann das Verhältnis des Außendurchmessers des dritten Hohlkörpers 21 zum Innendurchmesser des zweiten Hohlkörpers 3 vorzugsweise zwischen 1:4 und 1:2 liegen. Dadurch ist zwischen der Außenseite des dritten Hohlkörpers 21 und der Innenseite des zweiten Hohlkörpers 3 ein Zwischenraum 22 gebildet, wie dies aus Fig. 15 näher ersichtlich ist. Dieser Zwischenraum 22 ist, wie weiter unten noch näher ersichtlich wird, bei Abgabe einer Spülflüssigkeit durch den dritten Hohlkörper 21 in eine Körperöffnung oder-höhle eines Individuums als Rückflusskanal für aus der betreffenden Körperöffnung oder-höhle austretende gegebenenfalls Feststoffe mit sich führende Spülflüssigkeit gegebenenfalls unter Zuhilfenahme einer zusätzlichen Absaugvorrichtung nutzbar.

An seinem in Fig. 13 rechts dargestellten proximalen Ende ist der dritte Hohlkörper 21 mit dem bereits erwähnten Anlageteil 13 des Schiebemechanismus 11 verbunden. Der Anlageteil 13 ist als flacher Körper ausgebildet, der auf seinen äußeren Schmalseiten wippenartige elastische Betätigungsglieder 23, 24 aufweist, die zum in Fig. 13 links liegenden distalen Ende hin Rastelemente 25 bzw. 26 aufweisen. Diese Rastelemente 25 bzw. 26 lassen sich durch Druckausübung auf die Betätigungsglieder 23, 24 aus ihrer in Fig. 13 gezeigten Position anheben, um sodann die Rastelemente 25, 26 in passenden Aufnahmen 27 bzw. 28 des Aufnahmeteiles 12 zu verriegeln bzw. um eine solche Verriegelung wieder zu lösen. Diese Aufnahmen 27 und 28 sind aus Fig. 15 näher ersichtlich.

Der aus dem Aufnahmeteil 12 und dem Anlageteil 13 gebildete Schiebemechanismus 11 weist somit eine Verriegelungseinrichtung auf, die ein Verschieben des dritten Hohlkörpers 21 relativ zu dem zweiten Hohlkörper 3 lediglich bis zu einer Verriegelung des Anlageteiles 13 an oder in dem Aufnahmeteil 12 ermöglicht.

Der Anlageteil 13 weist wie bei der medizinischen Vorrichtung gemäß Fig. 1 bis 3 an seinem proximalen Ende ein einen Längsdurchgang aufweisendes Anschlussteil 14 auf, welches vorzugsweise ein Luer-Lock-Anschlussteil sein kann. Der Längsdurchgang dieses Anschlussteiles 14 ist mit dem durchgehenden Lumen des dritten Hohlkörpers 21 verbunden.

In Fig. 14 und 15 sind der in Fig. 12 dargestellte äußere zweite Hohlkörper 3 mit dem zum proximalen Ende hin trichterförmig offenen Aufnahmeteil 12 und der in Fig. 13 dargestellte dritte Hohlkörper 21 als innerer Hohlkörper mit dem Anlageteil 13 im zusammengesetzten Zustand als medizinische Vorrichtung 1' dargestellt, die gegenüber der medizinischen Vorrichtung gemäß Fig. 1 bis 3 zu einer Spülvorrichtung gemäß einer ersten Ausführungsform modifiziert ist. In diesem Zustand ist der Anlageteil 13 in dem Aufnahmeteil 12 hinsichtlich der Längsverschiebbarkeit verriegelt, wie dies deutlich aus der perspektivischen Schnittansicht gemäß Fig. 15 hervorgeht. Damit ist der dritte Hohlkörper 21 in seiner Längsverschiebbarkeit innerhalb des zweiten Hohlkörpers 3 festgelegt. Der dritte Hohlkörper 21 steht dabei mit seinem distalen Ende aus dem distalen Ende des zweiten Hohlkörpers 3 um eine gewisse Distanz vor. In der Praxis kann diese Distanz zwischen beispielsweise 1cm und 10cm liegen. In diesem Zustand kann eine Spülflüssigkeit durch den dritten Hohlkörper 21 in eine Körperöffnung oder-höhle eines Individuums eingeführt werden, in die zuvor die medizinische Vorrichtung gemäß Fig. 1 bis 3 eingeführt worden ist und deren erster Hohlkörper durch den dritten Hohlkörper 21 ersetzt bzw. ausgetauscht ist. Der zwischen der Außenseite des dritten Hohlkörpers 21 und der Innenseite des ersten Hohlkörpers 3 gebildete Zwischenraum 22 kann dann als Rückflusskanal für die aus der betreffenden Körperöffnung oder-höhle wieder austretende gegebenenfalls Feststoffe mit sich führende Spülflüssigkeit genutzt werden.

Der als Rückflusskanal dienende Zwischenraum 22 führt, wie aus Fig. 15 ersichtlich ist, in den Innenraum des Anlageteiles 12 hinein. Die durch diesen Rückflusskanal hindurch zurückgeleitete Spülflüssigkeit, die gegebenenfalls Feststoffe mit sich führt, kann dann von dem proximalen Endbereich der Spülvorrichtung 1' nach außen abfließen. Dieses Abfließen kann beispielsweise durch den nicht dichtenden Anlagebereich zwischen dem Aufnahmeteil 12 und dem Anlageteil 13 erfolgen und/oder durch gesonderte Abfließöffnungen in dem Aufnahmeteil 12 und/oder in dem Anlageteil 13. Diese Art des Abfließens der gegebenenfalls Feststoffe mit sich führenden Spülflüssigkeit aus dem Rückflusskanal kann übrigens in entsprechender Weise auch bei anderen Ausführungsformen der Erfindung angewandt sein, insbesondere bei den nachstehend noch näher beschriebenen beiden weiteren Ausführungsformen der Erfindung.

In Fig. 16 ist das in Fig.14 rechts dargestellte proximale Ende der medizinischen Vorrichtung in einer Draufsicht dargestellt. Dabei ist ersichtlich, dass die Aufnahmen 27 bzw. 28 in dem Aufnahmeteil 12 breiter sind als die Rastelemente 25 bzw. 26 des Anlageteiles 13. Dadurch kann das Anlageteil 13 quer zu seiner Längsrichtung relativ zu dem Aufnahmeteil 12 verschoben werden. Diese Verschiebbarkeit ist vorzugsweise erst nach erfolgter Verriegelung von Aufnahmeteil 12 und Anlageteil 13 gegeben. Grundsätzlich kann die betreffende Verschiebbarkeit zwischen Aufnahmeteil 12 und Anlageteil 13 auch vor deren Verriegelung gegeben sein. Durch das betreffende Verschieben kann dann der von dem zweiten Hohlkörper 3 aufgenommene dritte Hohlkörper 21 mit seiner Außenseite an die Innenseite des zweiten Hohlkörpers 3 herangeführt bzw. angelegt werden, um den Zwischenraum zwischen seiner Außenseite und der Innenseite des zweiten Hohlkörpers 3 einseitig zu vergrößern. Dies kann von besonderem Nutzen in dem Fall sein, dass die aus einer Körperöffnung oder-höhle eines Individuums austretende Spülflüssigkeit, die durch den dritten Hohlkörper 21 in diese zuvor eingeführt worden ist, größere Feststoffe zur Ableitung mit sich führt, die bei konzentrischer Anordnung des dritten Hohlkörpers 21 in dem zweiten Hohlkörper 3 nicht abgeleitet werden könnten.

Ergänzend ist in diesem Zusammenhang noch anzumerken, dass in Abweichung von dem vorstehend erläuterten Aufbau der an dem Aufnahmeteil 12 und dem Anlageteil 13 gebildeten Verriegelungseinrichtung die Positionen ihrer Rastelemente 25, 26 und ihrer Aufnahmen 27, 28 auch vertauscht sein können.

Mit der vorstehend beschriebenen medizinischen Vorrichtung 1' ist ein Verfahren zum Spülen einer Körperöffnung oder -höhle, wie einer Niere eines Individuums mittels einer durch den dritten Hohlkörper 21 in die betreffende Körperöffnung oder -höhle eingeleiteten Spülflüssigkeit ermöglicht. Die aus dieser Körperöffnung oder-höhle wieder austretende Spülflüssigkeit, die gegebenenfalls Feststoffe mit sich führt, wird durch den Zwischenraum 22 zwischen der Außenseite des dritten Hohlkörpers 21 und der Innenseite des zweiten Hohlkörpers 3 abgeleitet, gegebenenfalls durch Absaugen. Die vorliegende medizinische Vorrichtung kommt insbesondere als Spülvorrichtung 1' zur Anwendung, nachdem an Hand der Fig. 1 bis 3 beschriebene medizinische Vorrichtung in die zuvor erwähnte Körperöffnung oder -höhle eingeführt und deren erster Hohlkörper und die Formungseinrichtung in dem zweiten Hohlkörper 3 durch den hier beschriebenen dritten Hohlkörper 21 ersetzt worden sind. Die medizinische Vorrichtung 1' gemäß der vorliegenden Erfindung wird also dadurch gebildet, dass in der medizinischen Vorrichtung gemäß Fig. 1 bis 3 dessen erster länglicher Hohlkörper (2 in Fig. 2 und 3) und die Formungseinrichtung (6 in Fig. 4 bis 11) durch den hier beschriebenen dritten Hohlkörper 21 ausgetauscht oder ersetzt werden, je nachdem, ob die medizinische Vorrichtung gemäß Fig. 1 bis 3 bereits in eine Körperhöhle oder-öffnung eines Individuums eingeführt ist oder noch einzuführen ist.

Grundsätzlich kann das vorstehend erwähnte Verfahren zum Spülen einer Körperöffnung bzw. -höhle, wie einer Niere eines Individuums auch allein mittels der beschriebenen medizinischen Vorrichtung 1' ausgeführt werden, ohne dass diese zuvor als medizinische Vorrichtung 1 gemäß Fig. 1 bis 3 ausgebildet ist. Dies heißt, dass die medizinische Vorrichtung 1' von Hause aus auch allein einsetzbar ist, also ohne die bei der medizinische Vorrichtung 1 vorgesehenen zusätzlichen Elemente 2, 4 und 6.

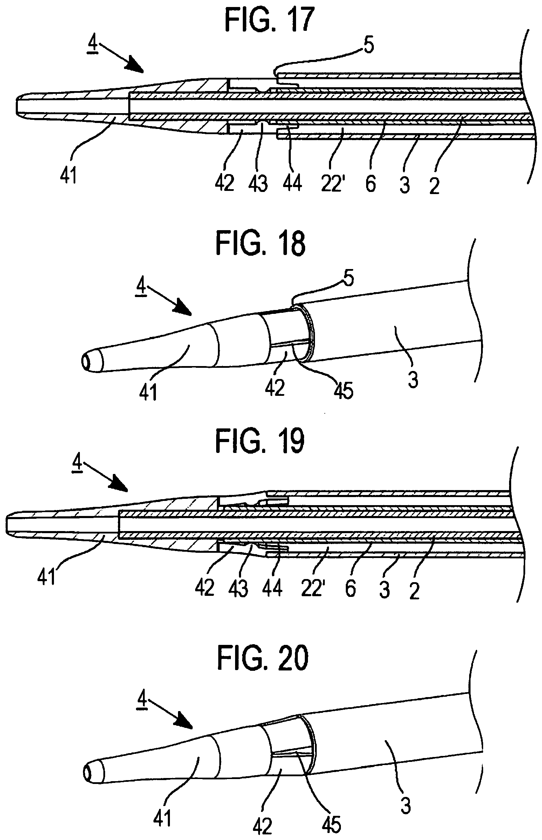

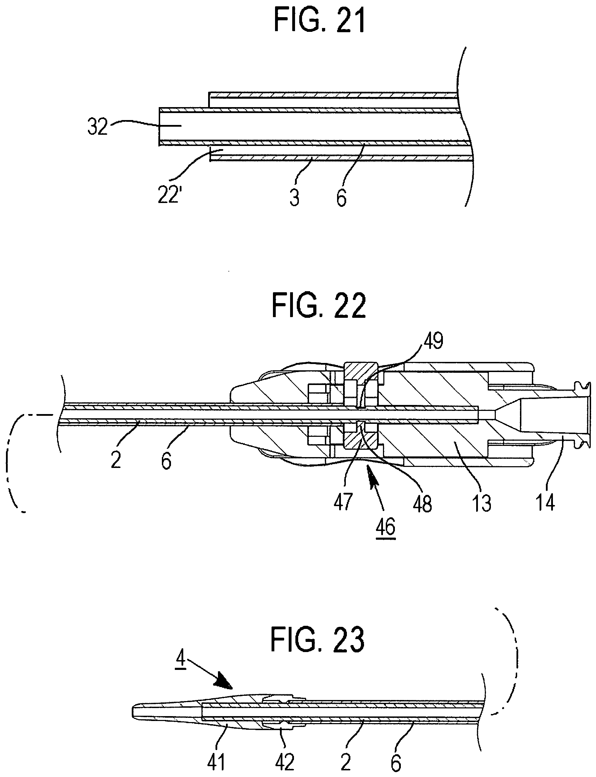

In Fig. 17 bis 20 ist eine weitere Variante des distalen Endbereichs der medizinischen Vorrichtung gemäß Fig. 1 bis 3 dargestellt, wobei diese modifizierte Vorrichtung mit diesem distalen Endbereich relativ einfach zu einer Spülvorrichtung gemäß einer zweiten Ausführungsform umgebildet werden kann, wie dies aus Fig.21 ersichtlich ist.

Gemäß Fig. 17 bis 20 besteht das mit dem inneren ersten Hohlkörper 2 verbundene (zum Beispiel verklebte) Spitzenteil 4 aus einem starren Spitzenteil 41 und einem sich daran zum proximalen Vorrichtungsende hin anschließenden radial aufweitbaren Übergangsteil 42. Dieser Übergangsteil 42 ist durch Verschieben der Formungseinrichtung 6 von einer ersten Stellung, in der er mit dem zweiten Hohlkörper 3 den stufenförmigen Übergang 5 (in Fig. 17 und 18 dargestellt) bildet, zu einer zweiten Stellung aufweitbar, in der er mit dem zweiten Hohlkörper 3 den stufenlosen Übergang (in Fig. 19 und 20 dargestellt) bildet. Der Spitzenteil 41 und der Übergangsteil 42 sind an ihren aneinander grenzenden Bereichen miteinander verbunden, beispielsweise durch eine Klebverbindung.

Der Übergangsteil 42 ist ein von dem Spitzenteil 41 aus trichterförmig aufweitbarer Teil mit einem innen liegenden Noppenbereich 43, durch dessen Berührung mittels der Formungseinrichtung 6 eine solche Aufweitung herbeiführbar ist (vergleiche die Fig. 17, 18 mit den Fig. 19, 20), dass der zunächst zwischen dem proximalen Ende des Übergangsteiles 42 und dem äußeren zweiten Hohlkörper 3 vorhandene stufenförmige Übergang 5 (siehe Fig. 17 und 18) in den zumindest weitgehend stufenlosen Übergang (siehe Fig. 19 und 20) überführbar ist. An seinem dem proximalen Vorrichtungsende (das ist in Fig. 17 bis 20 das jeweils rechts dargestellte Ende) zugewandten Ende weist der Übergangsteil 42 einen oder mehrere Anlageteile 44 auf, mit denen er bei seiner Aufweitung an der Innenwandung des zweiten Hohlkörpers 3 anliegt. Von diesem Anlageteil bzw. diesen Anlageteilen 44 erhebt sich von dem Anlageteil 44 ein äußerer Randbereich, dessen Höhe rechtwinklig zur Vorrichtungslängsrichtung und damit rechtwinklig zur Längsrichtung des zweiten Hohlkörpers 3 zumindest angenähert gleich dessen Wanddicke ist. Der Anlageteil 44 dient dreierlei Zwecken. Zum ersten dient es dazu zu verhindern, dass sich beim Zurückziehen des Hohlkörpers 2 (im Falle, dass dieser Hohlkörper ein Dilatator ist) in den Hohlkörper 3 (der als Schleuse dient) das Spitzenteil 4 an den distalen Enden des Hohlkörpers 3 nicht verhakt.

Zum Zweiten begrenzt der Anlageteil 44 unter anderem das Aufweiten des Spitzenteiles 4, und zum Dritten zentriert der Anlageteil 44 das Spitzenteil in dem vorzugsweise als Tubus ausgebildeten dritten Hohlkörper 3.

Im Umfangsbereich des Übergangsteiles 42 können ein oder mehr Schlitze 45 enthalten sein, um das Aufweiten des Übergangsteiles 42 zu erleichtern. Diese Schlitze 45 brauchen im Übrigen keine durchgehenden Öffnungen zwischen den an ihnen aneinander angrenzenden Materialbereichen des Übergangsteiles 42 zu sein. Vielmehr können die betreffenden Schlitze 45 durch Materialverdünnungen in dem das Übergangsteil 42 bildenden Material zwischen den an ihnen aneinander angrenzenden Materialbereichen gebildet sein.

Die Formungseinrichtung 6 ist im vorliegenden Fall durch ein längliches Rohr oder einen Schlauch mit einem inneren Lumen 32 und einer Außenabmessung in radialer Richtung gebildet, die kleiner ist als die Innenabmessung bzw. der Innendurchmesser des zweiten Hohlkörpers 3. Das Lumen 32 ist als Spülkanal zur Abgabe einer Spülflüssigkeit in eine Körperöffnung oder -höhle eines Individuums nutzbar, in die die medizinische Vorrichtung gemäß dem Hauptpatent mit dem zweiten Hohlkörper eingeführt ist. Zwischen der zuvor erwähnten Außenabmessung der Formungseinrichtung 6 und der Innenseite des zweiten Hohlkörpers 3 ist ein Zwischenraum 22' gebildet. Dieser Zwischenraum 22' ist als Rückflusskanal für eine Spülflüssigkeit nutzbar, welche nach Entfernen des ersten Hohlkörpers 2 samt seines Spitzenteiles 4 unter Freilegung dieses Zwischenraumes 22' zum distalen Vorrichtungsende hin als Spülkanal für die Abgabe einer Spülflüssigkeit in eine Körperöffnung oder -höhle eines Individuums dient.

Diese zuletzt erwähnte Konfiguration der medizinischen Vorrichtung als Spülvorrichtung ist in Fig.21 veranschaulicht. Dort sind am distalen Vorrichtungsende nach Entfernen des ersten Hohlkörpers 2 samt seines Spitzenteiles 4 unter Freilegung des erwähnten Zwischenraumes 22' lediglich die Formungseinrichtung 6 als inneres Hohlkörperelement mit dem Lumen 32 als Spülkanal und der zweite Hohlkörper 3 zu sehen, zwischen dem und der Formungseinrichtung 6 der Zwischenraum 22' als Rückflusskanal vorhanden ist.

In Fig.22 ist in einer nicht maßstäblichen Schnittansicht ein für den Einsatz in einem Aufnahmeteil (12 in Fig. 1 bis 3) am proximalen Ende der medizinischen Vorrichtung gemäß Fig. 1 bis 3 vorgesehener Anlageteil 13 mit dem ersten Hohlkörper 2 und der Formungseinrichtung 6 dargestellt. Der Hohlkörper 2 und die Formungseinrichtung 6 sind, wie durch eine Strichpunktlinie angedeutet, mit dem in Fig.23 dargestellten Spitzenteil 4 verbunden, welches den Spitzenteil 41 und den Übergangsteil 42 umfasst. Der zweite Hohlkörper 3 ist in Fig. 22 und 23 nicht dargestellt. Dieser zweite Hohlkörper 3 ist an einem Aufnahmeteil 12 angebracht, wie dies beispielsweise in der oben erläuterten Fig.12 gezeigt ist.

In dem Anlageteil 13 wird der erste Hohlkörper 2 am proximalen Vorrichtungsende mittels eines Verriegelungsmechanismus 46 lösbar festgehalten. Dieser Verriegelungsmechanismus 46 umfasst einen quer zur Vorrichtungslängsrichtung bewegbaren Schieber 47, der mit einem Verriegelungsvorsprung 48 in eine quer zur Vorrichtungslängsrichtung verlaufende Umfangsnut 49 des ersten Hohlkörpers 2 einführbar bzw. aus dieser herausführbar ist. Im eingeführten Zustand blockiert der Verriegelungsmechanismus 46 ein Herausziehen des ersten Hohlkörpers 2 aus dem Anlageteil 13; im herausgeführten Zustand gestattet der Verriegelungsmechanismus 46 indessen ein Herausziehen des ersten Hohlkörpers 2 aus dem Anlageteil 13. Zusammen mit diesem Herausziehen werden sodann auch das Spitzenteil 4 vom distalen Vorrichtungsende weggezogen, so dass schließlich dort lediglich der zweite Hohlkörper 3 und die Formungseinrichtung 6 verbleiben, wie dies in Fig.21 gezeigt ist.

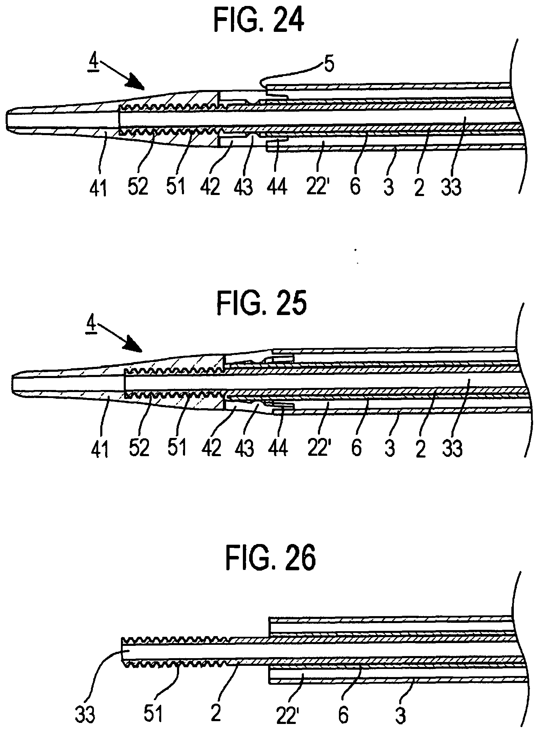

In Fig. 24 bis 26 ist eine noch weitere Variante des distalen Endbereichs der medizinischen Vorrichtung gemäß Fig. 1 bis 3 dargestellt, wobei die betreffende Vorrichtung mit diesem distalen Endbereich ebenfalls relativ leicht zu einer Spülvorrichtung gemäß einer dritten Ausführungsform umgebildet werden kann, wie dies aus Fig. 26 ersichtlich ist.

Die Fig. 24 und 25 zeigen in nicht maßstäblichen Schnittansichten einen gegenüber dem in Fig. 17 und 19 dargestellten distalen Endbereich der medizinischen Vorrichtung gemäß Fig. 1 bis 3 modifizierten distalen Endbereich. In den beiden Fig. 24 und 25 sind Elemente, die mit in Fig. 17 und 19 gezeigten Elementen übereinstimmen, mit denselben Bezugszeichen bezeichnet wie dort; die Formungseinrichtung 6 befindet sich analog zu den Fig. 17 und 20 in zwei unterschiedlichen Stellungen, einer ersten Stellung in Fig.24, in der der Übergangsbereich 5 zwischen dem äußeren zweiten Hohlkörper 3 und dem Übergangsteil 42 gebildet ist, und einer von der ersten Stellung verschiedenen zweiten Stellung in Fig. 25, in der der Übergangsbereich 5 zumindest weitgehend übergangslos gemacht ist.

Der in Fig. 24 und 25 dargestellte distale Endbereich unterscheidet sich von dem in Fig. 17 und 19 dargestellten distalen Endbereich lediglich dadurch, dass der Spitzenteil 41 und der innere erste Hohlkörper 2 durch eine lösbare Verbindungseinrichtung miteinander verbunden sind. Hinsichtlich der übrigen Elemente gelten für den distalen Endbereich gemäß Fig. 24 und 25 dieselben Ausführungen, die zu den Fig. 17 bis 19 gemacht worden sind.

Die zuvor erwähnte lösbare Verbindungseinrichtung ist hier durch eine Schraubverbindungseinrichtung gebildet, welche einen Schraubverbindungsteil 51 am distalen Außenumfang des ersten Hohlkörpers 2 und einen Mutternverbindungsteil 52 umfasst, der hier in dem Spitzenteil 4 enthalten ist. Durch Lösen dieser Schraubverbindungseinrichtung 51, 52 kann der Spitzenteil 41 zusammen mit dem Übergangsteil 42 von dem ersten Hohlkörper 2 entfernt werden, so dass der distale Endbereich dann so aussieht, wie dies in Fig. 26 gezeigt ist. Nach Entfernen allein des Spitzenteiles 4 unter Freilegung des Zwischenraumes 22' zwischen dem zweiten Hohlkörper 3 und der Formungseinrichtung 6 zum distalen Vorrichtungsende hin lässt sich das innere Lumen 33 des ersten Hohlkörpers 2 als Spülkanal für die Abgabe einer Spülflüssigkeit in eine Körperöffnung oder-höhle eines Individuums nutzen, in die zuvor die medizinische Vorrichtung gemäß Fig. 1 bis 3 eingeführt worden ist. Der genannte Zwischenraum 22' zwischen dem zweiten Hohlkörper 3 und der Formungseinrichtung 6 lässt sich dabei als Rückflusskanal für aus der betreffenden Körperöffnung oder -höhle austretende gegebenenfalls Feststoffe mit sich führende Spülflüssigkeit nutzen.

Es sei hier darauf hingewiesen, dass die zuvor erwähnte lösbare Verbindungseinrichtung auch in anderer Weise als hier beschrieben realisiert sein kann, beispielsweise durch eine Rastverbindungseinrichtung zwischen dem Spitzenteil 4 und dem ersten Hohlkörper 2.

Auch mit den vorstehend an Hand der Fig. 17 bis 23 sowie der Fig. 24 bis 26 beschriebenen zweiten und dritten Varianten der medizinischen Vorrichtung gemäß der Erfindung sind jeweils Verfahren zum Spülen einer Körperöffnung oder-höhle, wie einer Niere eines Individuums mittels einer durch die Formungseinrichtung 6 oder den ersten Hohlkörper 2 in die betreffende Körperöffnung oder -höhle eingeleiteten Spülflüssigkeit ermöglicht. Die aus dieser Körperöffnung oder-höhle wieder austretende Spülflüssigkeit, die gegebenenfalls Feststoffe mit sich führt, wird jeweils durch den Zwischenraum 22 bzw. 22' zwischen der Außenseite der Formungseinrichtung 6 und der Innenseite des zweiten Hohlkörpers 3 abgeleitet, gegebenenfalls durch Absaugen.

Die vorliegende medizinische Vorrichtung Fig. 17 bis 23 sowie gemäß Fig. 24 bis 26 kommt insbesondere zur Anwendung, nachdem die an Hand der Fig. 1 bis 3 beschriebene medizinische Vorrichtung 1 in die zuvor erwähnte Körperöffnung oder-höhle eingeführt und entweder deren erster Hohlkörper 2 samt seines Spitzenteiles 4 aus der Formungseinrichtung 6 oder lediglich das Spitzenteil 4 jeweils unter Freilegung des Zwischenraumes 22, 22' entfernt worden ist.

Grundsätzlich können auch die im Zusammenhang mit den Fig. 17 bis 23 und Fig. 24 bis 26 zuvor erwähnten Verfahren zum Spülen von Körperöffnungen bzw. - höhlen, wie Nieren von Individuen jeweils allein ausgeführt werden, ohne zuvor als medizinische Vorrichtungen 1 gemäß Fig. 1 bis 3 ausgebildet worden zu sind. Damit sind auch die entsprechend den Fig. 17 bis 23 bzw. Fig. 24 bis 26 ausgestalteten medizinischen Vorrichtungen 1' jeweils von Hause aus allein einsetzbar, also ohne die bei der medizinische Vorrichtung 1 vorgesehenen zusätzlichen Elemente 2, 4 und 6.

Abschließend sei hierzu noch folgendes angemerkt. Vor allem bei gleichzeitiger Abgabe einer Spülflüssigkeit in eine Körperöffnung oder-höhle eines Individuums und Rückführung der aus der betreffenden Körperöffnung oder -höhle austretenden, gegebenenfalls Feststoffe mit sich führenden Spülflüssigkeit ist es bei sämtlichen vorstehend beschriebenen Ausführungsformen der medizinischen Vorrichtung gemäß der Erfindung für einen einwandfreien Betrieb zumindest von Vorteil, wenn nicht sogar erforderlich, dass der Abstand zwischen dem distalen Ende des die Spülflüssigkeit jeweils abgebenden Hohlkörpers bzw. Hohlkörperelements und der Eintrittsstelle der aus der betreffenden Körperöffnung oder-höhle wieder abzuführenden Spülflüssigkeit in dem Rückflusskanal möglichst groß ist. Zweckmäßigerweise liegt dieser Abstand zumindest beim Einfachen und vorzugsweise zumindest beim Zweifachen der Außenabmessung bzw. des Außendurchmessers des zweiten Hohlkörpers 3.

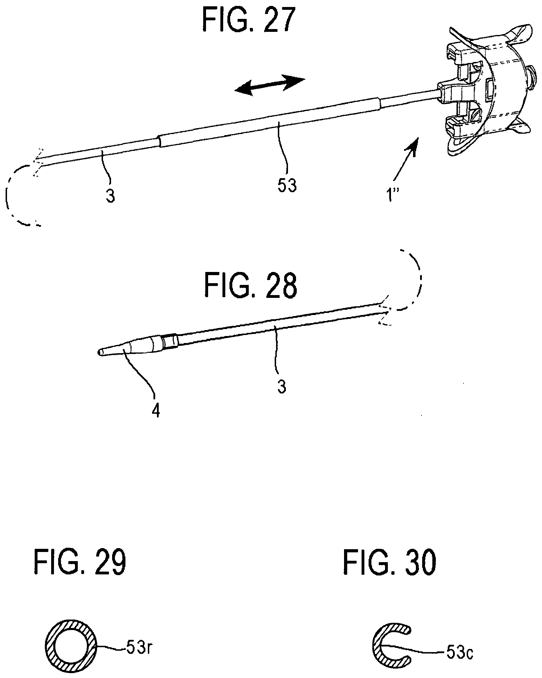

In Fig. 27 und 28 ist in einer durch eine Strichpunktlinie verbundenen, auseinander gezogenen Perspektivdarstellung in nicht maßstäblicher Größe eine mit 1" bezeichnete Abwandlung des in Fig. 1 gezeigten Ausführungsbeispiels der medizinischen Vorrichtung gemäß der Erfindung gezeigt. Gemäß Fig. 27 ist auf den zweiten Hohlkörper 3 ein Versteifungshohlkörper 53 aufgebracht, der beispielsweise ein Rohr oder Schlauch aus einem biokompatiblen Material sein kann. Durch diesen Versteifungshohlkörper 53 kann der medizinischen Vorrichtung 1" im Bereich ihrer beiden Hohlkörper, von denen in Fig. 27 und 28 lediglich der Hohlkörper 3 sichtbar ist, eine gegenüber der Steifigkeit dieser beiden Hohlkörper erhöhte Steifigkeit verliehen werden, wie sie zuweilen beim Gebrauch der betreffenden medizinischen Vorrichtung 1" erwünscht sein kann. Je nach Anwendungsfall können dabei Versteifungshohlkörper 53 mit unterschiedlichen Steifigkeiten verwendet werden.

Der Versteifungshohlkörper 53 kann in seinem Querschnitt unterschiedliche Formen aufweisen, wie sie beispielsweise in den beiden Schnittansichten gemäß Fig. 29 und Fig. 30 veranschaulicht sind.

Im Falle der in Fig. 29 dargestellten runden, geschlossenen Hohlquerschnittsform kann der mit 53r bezeichnete Versteifungshohlkörper vom distalen Ende der medizinischen Vorrichtung 1" auf deren zweiten Hohlkörper 3 aufgeschoben werden bzw. sein. Im Falle der in Fig. 30 dargestellten, mit 53c bezeichneten offenen C-förmigen Querschnittsform kann der Versteifungshohlkörper 53c auf den zweiten Hohlkörper 3 der medizinischen Vorrichtung 1 entweder ebenfalls aufgeschoben oder auf diesen aufgeklemmt werden.

Der jeweilige Versteifungshohlkörper 53 bzw. 53r oder 53c kann dabei entweder als einziges Element auf dem Hohlkörper 3 aufgebracht sein, oder er kann auf diesen in einer Mehrzahl, beispielsweise zweifach oder dreifach hintereinander aufgebracht sein. Dabei können die einzelnen auf den Hohlkörper hintereinander aufgebrachten Versteifungshohlkörper gleiche oder unterschiedliche Steifigkeit aufweisen. Überdies können Versteifungshohlkörper auch übereinander auf den Hohlkörper aufgebracht sein, entweder durch Aufschieben oder durch Aufklemmen im Falle der in Fig. 30 dargestellten Querschnittsform.

### Bezugszeichenliste

- 1, 1": medizinische Vorrichtung bzw. Dilatator
- 1': medizinische Vorrichtung bzw. Spülvorrichtung
- 2: erster Hohlkörper, Dilatatortubus
- 3: zweiter Hohlkörper, Außentubus
- 4: Spitzenteil
- 5: stufenförmiger Übergang
- 6, 6a, 6b: Formungsteil
- 7, 7a: Drückteil
- 8: Aufnahmeraum
- 9: stufenloser Übergang
- 10: Vertiefungsteil
- 11: Schiebemechanismus
- 12: Aufnahmeteil
- 13: Anlageteil
- 14: Anschlussteil
- 15: Innenteil
- 16: Außenteil
- 17: Gleitteil
- 18: Rastnasen
- 19: Rastnasenaufnahmen
- 20: Anschlag
- 21: dritter Hohlkörper
- 22, 22': Zwischenraum
- 23, 24: Betätigungsglied
- 25, 26: Rastelement
- 27, 28: Aufnahme
- 29: durchgehendes Lumen
- 32: Lumen, Spülkanal
- 33: Lumen, Spülkanal
- 41: Spitzenteil
- 42: Übergangsteil
- 43: Noppenbereich
- 44: Anlageteil(e)
- 45: Schlitz(e)
- 46: Verriegelungsmechanismus
- 47: Schieber
- 48: Verriegelungsvorsprung
- 49: Umfangsnut
- 51: Schraubverbind ungsteil
- 52: Mutternverbindungsteil
- 53, 53r, 53c: Versteifungshohlkörper

## Patentansprüche

1. Medizinische Vorrichtung (1) zum Einführen in eine Körperöffnung oder -höhle eines Individuums, mit einem ersten länglichen Hohlkörper (2) und einem zweiten länglichen Hohlkörper (3), in welchem der erste Hohlkörper (2) aufgenommen ist, wobei zwischen einem Ende des zweiten Hohlkörpers (3), aus dem der erste Hohlkörper (2) mit seinem zum Einführen in eine Körperöffnung oder-höhle eines Individuums vorgesehenen distalen Ende vorsteht, und diesem ersten Hohlkörper (2) ein in radialer Richtung verlaufender stufenförmiger Übergang (5) vorhanden ist, wobei das distale Ende des ersten Hohlkörpers (2) als Spitzenteil (4) ausgebildet ist oder ein Spitzenteil (4) aufweist, welches bis zu dem zweiten Hohlkörper (3) unter Bildung des stufenförmigen Übergangs (5) verläuft,
**dadurch gekennzeichnet,**
- **dass** zwischen dem Spitzenteil (4) und dem zweiten Hohlkörper (3) eine in deren Längsrichtung verschiebbare Formungseinrichtung (6, 7; 6, 10) aufgenommen ist
- und **dass** die Formungseinrichtung (6, 7; 6, 10) im Bereich des stufenförmigen Übergangs (5) so geformt ist, dass durch ihre Verschiebung in der betreffenden Längsrichtung das Spitzenteil (4) zu dem stufenförmigen Übergang (5) hin in radialer Richtung unter dessen zumindest weitgehender Aufhebung zu einem stufenlosen Übergang (9) aufweitbar und/oder der zweite Hohlkörper (3) zu dem stufenförmigen Übergang (5) hin in radialer Richtung unter dessen zumindest weitgehender Aufhebung zu einem stufenlosen Übergang (9) zusammenziehbar ist.

2. Medizinische Vorrichtung (1) nach Anspruch 1,
**dadurch gekennzeichnet, dass** die Formungseinrichtung (6, 7; 6, 10) ein Formungsteil (6) enthält, das auf seiner Außenseite an seinem zu dem stufenförmigen Übergang (5) zwischen dem Spitzenteil (4) und dem zweiten Hohlkörper (3) hin zu bewegenden Bereich einen Drückteil (7) aufweist, durch den im angrenzenden Bereich zwischen dem Spitzenteil (4) und dem zweiten Hohlkörper (3) der die in radialer Richtung kleinere Außenabmessung aufweisende Spitzenteil (4) bis zu der größeren Außenabmessung in radialer Richtung des zweiten Hohlkörpers (3) aufweitbar ist.

3. Medizinische Vorrichtung (1) nach Anspruch 1 oder 2,
**dadurch gekennzeichnet, dass** die Formungseinrichtung (6, 7; 6, 10) ein Formungsteil (6) enthält, das auf seiner Außenseite an seinem zu dem stufenförmigen Übergang (5) zwischen dem Spitzenteil (4) und dem zweiten Hohlkörper (3) hin zu bewegenden Bereich einen Vertiefungsteil (10) aufweist, durch den im angrenzenden Bereich zwischen dem Spitzenteil (4) und dem zweiten Hohlkörper (3) der die in radialer Richtung größere Außenabmessung aufweisende zweite Hohlkörper (3) bis zu dem die kleinere Außenabmessung in radialer Richtung aufweisenden Spitzenteil (4) absenkbar ist.

4. Medizinische Vorrichtung (1) nach einem der Ansprüche 1 bis 3,
**dadurch gekennzeichnet, dass** mit zumindest dem einen der beiden Hohlkörper (2, 3) und der Formungseinrichtung (6, 7; 6, 10) ein Schiebemechanismus (11) verbunden ist, durch dessen Betätigung die Formungseinrichtung (6, 7; 6, 10) relativ zu zumindest dem einen der beiden Hohlkörper (2,3) in dessen Längsrichtung verschiebbar ist.

5. Medizinische Vorrichtung (1) nach Anspruch 4,
**dadurch gekennzeichnet, dass** der Schiebemechanismus (11) aus einem mit dem einen der Hohlkörper (2, 3) verbundenen Aufnahmeteil (12) und einem mit der Formungseinrichtung (6, 7; 6, 10) verbundenen Anlageteil (13) besteht, welches von dem Aufnahmeteil (12) aufnehmbar und relativ zu diesem in der Hohlkörperlängsrichtung verschiebbar ist.

6. Medizinische Vorrichtung (1) nach Anspruch 5,
**dadurch gekennzeichnet, dass** der Schiebemechanismus (11) zwischen dem Aufnahmeteil (12) und dem Anlageteil (13) eine Verriegelungseinrichtung (18, 19) aufweist, die ein Verschieben der Formungseinrichtung (6, 7; 6, 10) relativ zu zumindest einem der Hohlkörper (2, 3) erst nach oder im Zuge einer Verriegelung des Anlageteiles (13) an oder in dem Aufnahmeteil (12) ermöglicht.

7. Medizinische Vorrichtung (1) nach einem der vorhergehenden Ansprüche,
**dadurch gekennzeichnet, dass** sie als medizinischer Dilatator (1) ausgebildet ist, an dessen für ein Einführen in eine Körperöffnung oder -höhle eines Individuums dienenden distalen Ende als erster Hohlkörper (2) ein als Spitzenteil (4) ausgebildeter oder mit dem Spitzenteil (4) verbundener Dilatatortubus (2) vorgesehen ist, der von einem Außentubus (3) als zweitem Hohlkörper (3) aufgenommen ist, zwischen dem und dem Dilatatortubus (2) die in Längsrichtung verschiebbare Formungseinrichtung (6, 7; 6, 10) aufgenommen ist.

8. Medizinische Vorrichtung (1) nach einem der vorhergehenden Ansprüche,
**dadurch gekennzeichnet,**
**dass** der erste Hohlkörper (2) und die Formungseinrichtung (6) durch einen dritten Hohlkörper (21) mit einer solchen Außenabmessung ersetzbar sind, zwischen der und der Innenabmessung des ersten Hohlkörpers (2) ein Zwischenraum (22) gebildet ist, und dass dieser Zwischenraum (22) bei Abgabe einer Spülflüssigkeit durch den dritten Hohlkörper (21) in eine Körperöffnung oder -höhle eines Individuums als Rückflusskanal für aus der betreffenden Körperöffnung oder -höhle austretende gegebenenfalls Feststoffe mit sich führende Spülflüssigkeit nutzbar ist.

9. Medizinische Vorrichtung (1) nach Anspruch 8,
**dadurch gekennzeichnet, dass** mit zumindest einem der den zweiten Hohlkörper (3) und den dritten Hohlkörper (21) umfassenden Hohlkörper (3, 21) ein Schiebemechanismus (11) verbunden ist, durch dessen Betätigung der dritte Hohlkörper (21) nach seinem Einführen in den zweiten Hohlkörper (3) in dessen Längsrichtung verschiebbar ist.

10. Medizinische Vorrichtung (1) nach Anspruch 9,
**dadurch gekennzeichnet, dass** der Schiebemechanismus (11) aus einem mit einem der den zweiten Hohlkörper (3) und den dritten Hohlkörper (21) umfassenden Hohlkörper (2, 21)verbundenen Aufnahmeteil (12) und einem mit dem anderen Hohlkörper verbundenen Anlageteil (13) besteht, welches von dem Aufnahmeteil (12) aufnehmbar und relativ zu diesem in der Hohlkörperlängsrichtung verschiebbar ist.

11. Medizinische Vorrichtung (1) nach Anspruch 10,
**dadurch gekennzeichnet, dass** der Schiebemechanismus (11) zwischen dem Aufnahmeteil (12) und dem Anlageteil (13) eine Verriegelungseinrichtung (18, 19) aufweist, die ein Verschieben des dritten Hohlkörpers (21) relativ zu dem ersten Hohlkörper (2) lediglich bis zu einer Verriegelung des Anlageteiles (13) an oder in dem Aufnahmeteil (12) ermöglicht.

12. Medizinische Vorrichtung nach einem der Ansprüche 1 bis 7,
**dadurch gekennzeichnet, dass**
- bei Bildung des Spitzenteiles (4) durch einen am distalen Vorrichtungsende vorgesehenen starren Spitzenteil (41) und einen sich daran zum proximalen Vorrichtungsende hin anschließenden radial aufweitbaren Übergangsteil (42), der durch Verschieben der Formungseinrichtung (6) von einer ersten Stellung, in der er mit dem zweiten Hohlkörper (3) den stufenförmigen Übergang (5) bildet, zu einer zweiten Stellung aufweitbar ist, in der er mit dem zweiten Hohlkörper (3) den stufenlosen Übergang bildet,
- und bei Vorhandensein eines Zwischenraumes (22') zwischen der Außenseite der Formungseinrichtung (6) und der Innenseite des zweiten Hohlkörpers (3)
der Innenraum der Formungseinrichtung (6) nach Entfernen des ersten Hohlkörpers (2) samt seines Spitzenteiles (4) unter Freilegung des genannten Zwischenraumes zum distalen Vorrichtungsende hin als Spülkanal für die Abgabe einer Spülflüssigkeit in eine Körperöffnung oder -höhle eines Individuums und der genannte Zwischenraum (22') als Rückflusskanal für aus der betreffenden Körperöffnung oder -höhle austretende gegebenenfalls Feststoffe mit sich führende Spülflüssigkeit nutzbar sind.

13. Medizinische Vorrichtung (1) nach Anspruch 12,
**dadurch gekennzeichnet, dass** der erste Hohlkörper (2) am proximalen Vorrichtungsende mittels eines Verriegelungsmechanismus (46) lösbar festgehalten ist.

14. Medizinische Vorrichtung nach einem der Ansprüche 1 bis 7,
**dadurch gekennzeichnet, dass**
- bei Bildung des Spitzenteiles (4) durch einen am distalen Vorrichtungsende vorgesehenen starren Spitzenteil (41) und einen sich daran zum proximalen Vorrichtungsende hin anschließenden radial aufweitbaren Übergangsteil (42), der durch Verschieben der Formungseinrichtung (6) von einer ersten Stellung, in der er mit dem zweiten Hohlkörper (3) den stufenförmigen Übergang (5) bildet, zu einer zweiten Stellung aufweitbar ist, in der er mit dem zweiten Hohlkörper (3) den stufenlosen Übergang bildet,
- und bei Vorhandensein eines Zwischenraumes (22') zwischen der Außenseite der Formungseinrichtung (6) und der Innenseite des zweiten Hohlkörpers (3)
der Innenraum des ersten Hohlkörpers (2) nach Entfernen allein des Spitzenteiles (4) unter Freilegung des genannten Zwischenraumes zum distalen Vorrichtungsende hin als Spülkanal für die Abgabe einer Spülflüssigkeit in eine Körperöffnung oder-höhle eines Individuums und der genannte Zwischenraum (22') als Rückflusskanal für aus der betreffenden Körperöffnung oder -höhle austretende gegebenenfalls Feststoffe mit sich führende Spülflüssigkeit nutzbar sind.

15. Medizinische Vorrichtung (1) nach Anspruch 14,
**dadurch gekennzeichnet, dass** das Spitzenteil (4)mit dem ersten Hohlkörper (2) durch eine lösbare Verbindungseinrichtung (51, 52) verbunden ist.

## Claims

1. Medical device (1) for introduction into a bodily orifice or cavity of an individual, having a first elongated hollow body (2) and a second elongated hollow body (3), in which the first hollow body (2) is accommodated, wherein between one end of the second hollow body (3), from which the first hollow body (2) projects with its distal end provided for introduction into a bodily orifice or cavity of an individual, and this first hollow body (2) a step-shaped transition (5) extending in radial direction is present, wherein the distal end of the first hollow body (2) is configured as tip part (4) or comprises a tip part (4), which extends to the second hollow body (3), forming the step-shaped transition (5),
**characterized in that**
- between the tip part (4) and the second hollow body (3) a shaping device (6, 7; 6, 10) that can be displaced in its longitudinal direction is accommodated
- and the shaping device (6, 7; 6, 10) in the region of the step-shaped transition (5) has such a shape that by its displacement in the corresponding longitudinal direction the tip part (4) toward the step-shaped transition (5) in radial direction, whilst cancelling it out, at least to a great extent, is expandable into a step-free transition (9), and/or the second hollow body (3) toward the step-shaped transition (5) in radial direction, whilst cancelling it out, at least to a great extent, is contractable into a step-free transition (9).

2. Medical device (1) according to claim 1,
**characterized in that**
the shaping device (6, 7; 6, 10) contains a shaping part (6) that on its outside in its region to be moved toward the step-shaped transition (5) between the tip part (4) and the second hollow body (3) has a pressing part (7), by which in the adjoining region between the tip part (4) and the second hollow part (3) the tip part (4) having the smaller outside dimension in the radial direction is expandable to the greater outside dimension in the radial direction of the second hollow body (3).

3. Medical device (1) according to claim 1 or 2,
**characterized in that**
the shaping device (6, 7; 6, 10) comprises a shaping part (6) that has a depression part (10) on its outside in its region to be moved toward the step-shaped transition (5) between the tip part (4) and the second hollow body (3), by which in the adjacent region between the tip part (4) and the second hollow body (3) the second hollow body (3) having the greater outside dimension in the radial direction can be lowered all the way to the tip part (4) having the smaller outside dimension in the radial direction.

4. Medical device (1) according to any one of claims 1 to 3,
**characterized in that**
with at least the one of the two hollow bodies (2, 3) and the shaping device (6, 7; 6, 10) a slide mechanism (11) is connected, by the activation of which the shaping device (6, 7; 6, 10) can be displaced relative to at least the one of the two hollow bodies (2, 3) in its longitudinal direction.

5. Medical device (1) according to claim 4,
**characterized in that**
the slide mechanism (11) consists of an accommodation part (12), which is connected with the one of the hollow bodies (2, 3), and a contact part (13), which is connected with the shaping device (6, 7; 6, 10) and which can be accommodated by the accommodation part (12) and can be displaced relative to same in the longitudinal direction of the hollow body.

6. Medical device (1) according to claim 5,
**characterized in that**
the slide mechanism (11) between the accommodation part (12) and the contact part (13) has a locking device (18, 19) that allows displacement of the shaping device (6, 7; 6, 10) relative to at least one of the hollow bodies (2, 3), only after or during the course of a locking of the contact part (13) on or in the accommodation part (12).

7. Medical device (1) according to any one of the preceding claims,
**characterized in that**
it is configured as a medical dilator (1), at the distal end of which, which serves for an introduction into a bodily orifice or cavity of an individual, as first hollow body (2) a dilator tube (2) configured as tip part (4) or connected with the tip part (4) is provided, which is accommodated by an outer tube (3) as the second hollow body (3), between which and the dilator tube (2) the shaping device (6, 7; 6, 10), which can be displaced in the longitudinal direction, is accommodated.

8. Medical device (1) according to any one of the preceding claims,
**characterized in that**
the first hollow body (2) and the shaping device (6) are replaceable by a third hollow body (21) with such outside diameter, between which and the inside dimension of the first hollow body (2) an interstice (22) is formed, and that this interstice (22) upon dispensing a flushing fluid through the third hollow body (21) into a bodily orifice or cavity of an individual can be used as return flow channel for flushing fluid exiting from the bodily orifice or cavity in question, which might be carrying solids with it.

9. Medical device (1) according to claim 8,
**characterized in that**
with at least one of the hollow bodies (3, 21) comprising the second hollow body (3) and the third hollow body (21) a slide mechanism (11) is connected, by the activation of which the third hollow body (21) after its introduction into the second hollow body (3) can be displaced in its longitudinal direction.

10. Medical device (1) according to claim 9,
**characterized in that**
the slide mechanism (11) consists of an accommodation part (12) connected with one of the hollow bodies (2, 21) comprising the second hollow body (3) and the third hollow body (21) and a contact part (13) connected with the other hollow body, which can be accommodated by the accommodation part (12) and can be displaced relative thereto in the longitudinal direction of the hollow body.

11. Medical device (1) according to claim 10,
**characterized in that**
the slide mechanism (11) comprises a locking device (18, 19) between the accommodation part (12) and the contact part (13), which facilitates a displacing of the third hollow body (21) relative to the first hollow body (2) only until the contact part (13) locks onto or into the accommodation part (12).

12. Medical device according to any one of claims 1 to 7,
**characterized in that**
- when the tip part (4) is formed by a rigid tip part (41) provided at the distal device end and a radially expandable transition part (42), which adjoins towards the proximal device end and is expandable by displacing the shaping device (6) from a first position, in which it forms the step-shaped transition (5) with the second hollow body (3), into a second position, in which it forms the step-free transition with the second hollow body (3),
- and in the case of the presence of an interstice (22') between the outside of the shaping device (6) and the inside of the second hollow body (3)
the interior of the shaping device (6) upon removal of the first hollow body (2) including its tip part (4) whilst uncovering the named interstice toward the distal device end can be used as flushing channel for dispensing a flushing fluid into a bodily orifice or cavity of an individual and the named interstice (22') as return flow channel for flushing fluid exiting the bodily orifice or cavity, which might be carrying solids with it.

13. Medical device (1) according to claim 12,
**characterized in that**
the first hollow body (2) is releasably retained on the proximal device end by a locking mechanism (46).

14. Medical device (1) according to any one of claims 1 to 7,
**characterized in that**
- when the tip part (4) is formed by a rigid tip part (41) provided at the distal device end and a radially expandable transition part (42), which adjoins toward the proximal device end and is expandable by displacing the shaping device (6) from a first position, in which it forms the step-shaped transition (5) with the second hollow body (3), into a second position, in which it forms the step-free transition with the second hollow body (3),
- and in the case of the presence of an interstice (22') between the outside of the shaping device (6) and the inside of the second hollow body (3)
the interior of the first hollow body (2) upon removal of the tip part (4) alone whilst uncovering the named interstice toward the distal device end can be used as flushing channel for the dispensing of a flushing fluid into a bodily orifice or cavity of an individual and the named interstice (22') as return flow channel for flushing fluid exiting the bodily orifice or cavity in question, which might be carrying solids with it.

15. Medical device (1) according to claim 14,
**characterized in that**
the tip part (4) is connected with the first hollow body (2) by a releasable connection device (51, 52).

## Revendications

1. Appareil médical (1), destiné à être introduit dans un orifice ou une cavité du corps d'un individu, avec un premier corps creux longitudinal (2) et un deuxième corps creux longitudinal (3), dans lequel est logé le premier corps creux (2), un passage étagé (5), évoluant dans le sens radial, existant entre une extrémité du deuxième corps creux (3), hors duquel le premier corps creux (2) fait saillie avec son extrémité distale, prévue pour l'introduction dans un orifice ou une cavité du corps d'un individu et ce premier corps creux (2), l'extrémité distale du premier corps creux (2) étant constituée en tant que partie formant pointe (4) ou présentant une partie formant pointe (4), laquelle évolue jusqu'au deuxième corps creux (3), en formant le passage étagé (5),
**caractérisé en ce**
- **qu'**un dispositif de formage (6, 7 ; 6, 10), déplaçable dans son sens longitudinal, est logé entre la partie formant pointe (4) et le deuxième corps creux (3) et que
- le dispositif de formage (6, 7 ; 6, 10) est formé, dans le secteur du passage étagé (5), de telle sorte que la partie formant pointe (4) peut être élargie, du fait de son déplacement dans le sens longitudinal concerné, par rapport au passage étagé (5), dans le sens radial, avec au moins sa large suppression par rapport à un passage continu (9) et / ou que le deuxième corps creux (3) peut être contracté par rapport au passage étagé (5), dans le sens radial, avec au moins sa large suppression par rapport à un passage continu (9).

2. Appareil médical (1) selon la revendication 1,
**caractérisé en ce que**
le dispositif de formage (6, 7 ; 6, 10) contient une pièce de formage (6), qui présente, sur son côté extérieur, une partie formant pression (7), sur sa zone à déplacer par rapport au passage étagé (5), entre la partie formant pointe (4) et le deuxième corps creux (3), à travers lequel la partie formant pointe (4), qui présente la dimension extérieure plus petite dans le sens radial, peut être élargie jusqu'à la dimension extérieure plus grande, dans le sens radial du deuxième corps creux (3) dans la zone adjacente entre la partie formant pointe (4) et le deuxième corps creux (3).

3. Appareil médical (1) selon la revendication 1 ou 2,
**caractérisé en ce que**
le dispositif de formage (6, 7 ; 6, 10) contient une pièce de formage (6), qui présente, sur son côté extérieur, sur sa zone, à déplacer par rapport au passage étagé (5), entre la partie formant pointe (4) et le deuxième corps creux (3), une partie formant évidement (10), à travers laquelle le deuxième corps creux (3), présentant la dimension extérieure plus grande dans le sens radial, peut être abaissé, dans la zone adjacente entre la partie formant pointe (4) et le deuxième corps creux (3), jusqu'à la partie formant pointe (4), présentant la dimension extérieure plus petite dans le sens radial.

4. Appareil médical (1) selon l'une des revendications 1 à 3,
**caractérisé en ce**
**qu'**au moins un mécanisme coulissant (11) est relié à au moins l'un des deux corps creux (2, 3) et au dispositif de formage (6, 7 ; 6, 10), par l'actionnement duquel le dispositif de formage (6, 7 ; 6, 10) peut être déplacé par rapport à au moins l'un des deux corps creux (2, 3) dans son sens longitudinal.

5. Appareil médical (1) selon la revendication 4,
**caractérisé en ce que**
le mécanisme coulissant (11) se compose d'une partie formant logement (12), reliée à l'un des corps creux (2, 3) et d'une partie formant appui (13), reliée au dispositif de formage (6, 7 ; 6, 10), lequel peut être logé par la partie formant logement (12) et peut être déplacé par rapport à celle-ci dans le sens longitudinal du corps creux.

6. Appareil médical (1) selon la revendication 5,
**caractérisé en ce que**
le mécanisme coulissant (11) présente, entre la partie formant logement (12) et la partie formant appui (13), un dispositif de verrouillage (18, 19), qui permet un déplacement du dispositif de formage (6, 7 ; 6, 10) par rapport à au moins l'un des corps creux (2, 3) seulement après ou au cours d'un verrouillage de la partie formant appui (13), contre ou dans la partie formant logement (12).

7. Appareil médical (1) selon l'une des revendications précédentes,
**caractérisé en ce**
**qu'**il est conçu, en tant que dilatateur médical (1), qu'il est prévu, à son extrémité distale, en tant que premier corps creux (2), servant pour une introduction dans un orifice ou une cavité du corps d'un individu, un tube formant dilatateur (2), conçu comme partie formant pointe (4) ou relié à la partie formant pointe (4), qui est logé par un tube extérieur (3) comme deuxième corps creux (3), le dispositif de formage (6, 7 ; 6, 10), déplaçable dans le sens longitudinal, étant logé entre le tube extérieur (3) et le tube formant dilatateur (2).

8. Appareil médical (1) selon l'une des revendications précédentes,
**caractérisé en ce que**
le premier corps creux (2) et le dispositif de formage (6) peuvent être remplacés par un troisième corps creux (21) avec une telle dimension extérieure, qu'un espace intermédiaire (22) est formé entre la dimension extérieure et la dimension intérieure du premier corps creux (2) et que cet espace intermédiaire (22) peut être utilisé, lors de la délivrance d'un liquide de lavage à travers le troisième corps creux (21) dans un orifice ou une cavité du corps d'un individu, en tant que canal de reflux pour des matières solides, qui sortent éventuellement de l'orifice ou de la cavité du corps concerné(e), avec le liquide de lavage, entraîné avec elles.

9. Appareil médical (1) selon la revendication 8,
**caractérisé en ce**
**qu'**un mécanisme coulissant (11) est relié à au moins l'un des corps creux (3, 21), comprenant le deuxième corps creux (3) et le troisième corps creux (21), par le biais de l'actionnement duquel le troisième corps creux (21) peut être déplacé, après son introduction dans le deuxième corps creux (3), dans son sens longitudinal.

10. Appareil médical (1) selon la revendication 9,
**caractérisé en ce que**
le mécanisme coulissant (11) se compose d'une partie formant logement (12), reliée à l'un des corps creux (2, 21), comprenant le deuxième corps creux (3) et le troisième corps creux (21) et d'une partie formant appui (13), reliée à l'autre corps creux, laquelle peut être logée par la partie formant logement (12) et peut être déplacée par rapport à celle-ci dans le sens longitudinal du corps creux.

11. Appareil médical (1) selon la revendication 10,
**caractérisé en ce que**
le mécanisme coulissant (11) présente un dispositif de verrouillage (18, 19) entre la partie formant logement (12) et la partie formant appui (13), qui permet un déplacement du troisième corps creux (21) par rapport au premier corps creux (2) uniquement jusqu'à un verrouillage de la partie formant appui (13) sur ou dans la partie formant logement (12).

12. Appareil médical selon l'une des revendications 1 à 7,
**caractérisé en ce que**,
- lors de la formation de la partie formant pointe (4) par une partie formant pointe rigide (41), prévue à l'extrémité distale de l'appareil et d'une partie formant passage (42), s'y raccordant par rapport à l'extrémité proximale de l'appareil et s'élargissant radialement, qui peut s'élargir, par déplacement du dispositif de formage (6) depuis une première position, dans laquelle il forme le passage étagé (5) avec le deuxième corps creux (3), vers une seconde position, dans laquelle il forme le passage continu avec le deuxième corps creux (3) et
- en cas d'existence d'un espace intermédiaire (22') entre le côté extérieur du dispositif de formage (6) et le côté intérieur du deuxième corps creux (3),
l'espace intérieur du dispositif de formage (6) peut être utilisé, après enlèvement du premier corps creux (2), y compris sa partie formant pointe (4), en dégageant l'espace intermédiaire nommé par rapport à l'extrémité distale de l'appareil, en tant que canal de lavage pour la délivrance d'un liquide de lavage dans un orifice ou une cavité du corps d'un individu et l'espace intermédiaire (22') nommé, peut être utilisé en tant que canal de reflux pour des matières solides, sortant le cas échéant de l'orifice ou de la cavité du corps concerné(e), avec le liquide de lavage, entraîné avec elles.

13. Appareil médical (1) selon la revendication 12,
**caractérisé en ce que**
le premier corps creux (2) est maintenu de manière amovible sur l'extrémité proximale de l'appareil au moyen d'un mécanisme de verrouillage (46).

14. Appareil médical (1) selon l'une des revendications 1 à 7,
**caractérisé en ce que**,
- lors de la formation de la partie formant pointe (4) par une partie formant pointe rigide (41), prévue à l'extrémité distale de l'appareil et une partie formant passage (42), s'y raccordant par rapport à l'extrémité proximale de l'appareil et s'élargissant radialement, qui peut s'élargir, par déplacement du dispositif de formage (6) depuis une première position, dans laquelle il forme le passage étagé (5) avec le deuxième corps creux (3), vers une seconde position, dans laquelle il forme le passage continu avec le deuxième corps creux (3) et
- en cas d'existence d'un espace intermédiaire (22') entre le côté extérieur du dispositif de formage (6) et le côté intérieur du deuxième corps creux (3),
l'espace intérieur du premier corps creux (2) peut être utilisé, après enlèvement de la seule partie formant pointe (4), en dégageant l'espace intermédiaire nommé par rapport à l'extrémité distale de l'appareil, en tant que canal de lavage, pour la délivrance d'un liquide de lavage dans un orifice ou une cavité du corps d'un individu et l'espace intermédiaire (22') nommé, peut être utilisé en tant que canal de reflux pour des matières solides, sortant le cas échéant de l'orifice ou de la cavité du corps concerné(e), avec le liquide de lavage, entraîné avec elles.

15. Appareil médical (1) selon la revendication 14;
**caractérisé en ce que**
la partie formant pointe (4) est reliée au premier corps creux (2) par le biais d'un dispositif de liaison amovible (51, 52).
